# EUROPÄISCHE PATENTSCHRIFT

(11) **EP 4 308 210 B1**
(45) Veröffentlichungstag und Bekanntmachung des Hinweises auf die Patenterteilung: **25.12.2024**
(21) Anmeldenummer: 22715556.1
(22) Anmeldetag: 15.03.2022
(51) Int. Cl.: A61M 16/20, F16K 27/00

(54) **MODULARE UMSCHALTVENTILBAUGRUPPE FÜR EINE BEATMUNGSVORRICHTUNG**
MODULAR SWITCHING-VALVE ASSEMBLY FOR A RESPIRATORY DEVICE
ENSEMBLE SOUPAPE DE COMMUTATION MODULAIRE POUR UN APPAREIL RESPIRATOIRE

(30) Priorität: 19.03.2021 DE 102021106784
(43) Veröffentlichungstag der Anmeldung: 24.01.2024
(73) Patentinhaber: Hamilton Medical AG, 7402 Bonaduz (CH)
(72) Erfinder: HEPTING, Daniel, 7026 Maladers (CH)
(74) Vertreter: Ruttensperger Lachnit Trossin Gomoll
(86) Internationale Anmeldenummer: PCT/EP2022/056638
(87) Internationale Veröffentlichungsnummer: WO 2022/194835

(56) Entgegenhaltungen:
- EP-A1- 2 857 735
- WO-A2-2008/157242
- DE-A1- 102007 047 699
- DE-A1- 2 918 791
- US-A- 5 020 570
- US-A- 5 605 179
- US-A- 5 749 562
- US-A1- 2020 173 571

## Beschreibung

Die vorliegende Erfindung betrifft eine Umschaltventilbaugruppe, zum Umschalten zwischen zwei Atemgasquellen einer Beatmungsvorrichtung, wobei die Umschaltventilbaugruppe umfasst:
- ein Gehäuse mit einer ersten Quellen-Anschlussöffnung zum Anschluss einer ersten Atemgasquelle, mit einer räumlich mit Abstand von der ersten angeordneten zweiten Quellen-Anschlussöffnung zum Anschluss einer zweiten Atemgasquelle und mit einer räumlich mit Abstand von der ersten und der zweiten Quellen-Anschlussöffnung angeordneten Ausgabe-Anschlussöffnung zur Ausgabe von Atemgas von wenigstens einer der Quellen-Anschlussöffnungen,
- eine erste Gaslieferleitung, welche die erste Quellen-Anschlussöffnung mit der Ausgabe-Anschlussöffnung verbindet,
- eine zweite Gaslieferleitung, welche die zweite Quellen-Anschlussöffnung mit der Ausgabe-Anschlussöffnung verbindet,
- eine im Gehäuse aufgenommene Ventilanordnung, welche verstellbar ist zwischen einem ersten Betriebszustand, in welchem sie die erste Gaslieferleitung zur Durchströmung mit Atemgas freigibt und die zweite Gaslieferleitung sperrt, und einem zweiten Betriebszustand, in welchem sie die erste Gaslieferleitung sperrt und die zweite Gaslieferleitung zur Durchströmung mit Atemgas freigibt.

Eine Umschaltventilbaugruppe mit den eingangs genannten Merkmalen ist aus der DE 29 18 791 A1 bekannt. Diese bekannte Umschaltventilbaugruppe dient dazu, einen an die Ausgabe-Anschlussöffnung angeschlossenen, nicht näher bezeichneten Verbraucher unterbrechungsfrei mit Gas aus Flaschenbatterien zu versorgen, welche an die erste und die zweite Quellen-Anschlussöffnung angeschlossen sind.

Besonders bei der Versorgung von künstlich beatmeten Patienten mit Atemgas kann eine unterbrechungsfreie Versorgung erheblich zum Behandlungserfolg beitragen oder sogar lebensrettend sein.

Eine unterbrechungsfreie Atemgasversorgung ist vor allem dann nicht selbstverständlich, wenn das Atemgas nicht einer quasi-unendlich großen Quelle, wie beispielsweise der Umgebungsatmosphäre, entnommen werden kann, sondern wenn das Atemgas aus einem begrenzten Vorrat stammt. Dies ist bei sogenannten "Hi-Flow" Beatmungsgeräten der Fall, welche unabhängig vom Atemrhythmus und vom Atemvolumen der einzelnen Atemhübe des Patienten Atemgas kontinuierlich zu dessen Mund oder/und Nase liefern. Das Atemgas, das häufig zu einer vom Patienten getragenen Nasenbrille oder Mund-Nasen-Maske geliefert wird, ist dann bevorzugt Sauerstoff oder mit Sauerstoff angereicherte Luft, welche einen höheren Sauerstoffgehalt als die Umgebungsatmosphäre aufweist.

Die aus der DE 29 18 791 A1 bekannte Umschaltventilbaugruppe lehrt ganz allgemein, die Gasdrücke der an den Quellen-Anschlussöffnungen angeschlossenen Gasquellen zu nutzen, um die Ventilanordnung selbsttätig zwischen ihrem ersten und ihrem zweiten Betriebszustand hin und her zu schalten. Dann also, wenn die Gasquelle an einer Quellen-Anschlussöffnung inhaltlich zur Neige geht, während die Gasquelle der jeweils anderen Quellen-Anschlussöffnung noch vollständig zur Verfügung steht, führt das Erschöpfen der einen Gasquelle zu einem abnehmenden Gasdruck in der mit dieser Gasquelle verbundenen Gaslieferleitung, sodass der dann relativ zum Gasdruck der einen Gasquelle größer werdende Gasdruck der anderen Gasquelle eine Verstellung der Ventilanordnung bewirkt. Hierdurch wird die zur Neige gehende Gasquelle strömungsmechanisch von der Ausgabe-Anschlussöffnung getrennt und die volle Gasquelle liefert an die Ausgabe-Anschlussöffnung. Die erschöpfte Gasquelle kann dann gegen eine frische ausgetauscht werden die dann durch ihren relativ größer werdenden Gasdruck aktiviert wird, wenn die zuletzt aktivierte Gasquelle ebenfalls beginnt, zur Neige zu gehen.

Die DE 29 18 791 A1 lehrt, an jede Quellen-Anschlussöffnung nicht nur eine Gasflasche als Vorrat, sondern ganze Flaschenbatterien mit einer Mehrzahl von Gasflaschen anzuschließen, um die Austauschzyklen der einzelnen an den Quellen-Anschlussöffnungen angeschlossenen Gasquellen möglichst lang zu gestalten.

Dies führt zu zweierlei Nachteilen: erstens führt die Verwendung von Flaschenbatterien zu einer verhältnismäßig schlechten Ausnutzung der einzelnen Flaschenfüllung, da die Flaschenbatterien einer Gasquelle untereinander über Fächerleitungen miteinander verbunden sein müssen, damit jede einzelne Flasche der Flaschenbatterien mit der zugeordneten Quellen-Anschlussöffnung verbunden ist und an diese Gas liefern kann. Hierdurch wird auf Seiten der Gasquelle der Totraum erhöht. Gas aus den Flaschen einer Flaschenbatterie kann zwischen den Flaschen der Flaschenbatterie strömen und muss nicht notwendigerweise zur Ausgabe-Anschlussöffnung gelangen.

Zweitens wird die mit Flaschenbatterien bestückte Umschaltventilbaugruppe schnell unhandlich und kann nur noch als stationäre Umschaltventilbaugruppe verwendet werden. Eine Konfiguration der Umschaltventilbaugruppe auf eine bestimmte Anzahl an daran anschließbaren Flaschen kann nur über eine entsprechende konstruktive Ausgestaltung der Umschaltventilbaugruppe durch entsprechende Verwendung von Fächerleitungen erfolgen, welche die gewünschte Anzahl an Flaschen mit einer Quellen-Anschlussöffnung verbinden. Eine Änderung einer einmal gewählten Konfiguration ist dann nur durch unerwünscht aufwändige Montagearbeit zu erreichen.

Angesichts der oben beschriebenen Nachteile ist es Aufgabe der vorliegenden Erfindung, die eingangs genannte Umschaltventilbaugruppe derart zu verbessern, dass mit ihr eine beliebige Anzahl an gesonderten Gasquellen, insbesondere Atemgasquellen, an einen Verbraucher, bevorzugt an eine Beatmungsvorrichtung, besonders bevorzugt an eine oben genannte "Hi-Flow"-Beatmungsvorrichtung, angeschlossen werden können, wobei die einzelnen Gasquellen zuverlässig entleerbar sein sollen und ein mit mehr als zwei Gasquellen versorgter Verbraucher weiterhin als mobile Umschaltventilbaugruppe nutzbar sein soll.

Die vorliegende Erfindung löst diese Aufgabe an der eingangs genannten Umschaltventilbaugruppe dadurch, dass die Umschaltventilbaugruppe als modulare Umschaltventilbaugruppe stapelbar ausgebildet ist, indem das Gehäuse einen ersten Außenseitenabschnitt mit einem ersten Stapel-Außenflächenabschnitt und einen relativ zum ersten Außenseitenabschnitt mit Abstand oder/und mit Winkelabstand angeordneten zweiten Außenseitenabschnitt mit einem zum ersten Stapel-Außenflächenabschnitt komplementär ausgebildeten zweiten Stapel-Außenflächenabschnitt aufweist, wobei die Ausgabe-Anschlussöffnung an einem Außenseitenabschnitt aus dem ersten und dem zweiten Außenseitenabschnitt ausgebildet ist, und wobei eine Quellen-Anschlussöffnung aus der ersten und der zweiten Quellen-Anschlussöffnung als Folge-Anschlussöffnung an dem jeweils anderen Außenseitenabschnitt aus dem ersten und dem zweiten Außenseitenabschnitt ausgebildet ist, und zwar derart, dass dann, wenn der erste und der zweite Stapel-Außenflächenabschnitt als Stapel-Außenflächenabschnitte gleichartiger gesonderter modularer Umschaltventilbaugruppen in Stapeleingriff miteinander gedacht sind, von der Auslass- und der Folge-Anschlussöffnung ausgehend sich jeweils ins Gehäuse hinein erstreckende Abschnitte von Gaslieferleitungen zu einem kontinuierlichen Gaslieferleitungsbereich verbunden sind.

Die Umschaltventilbaugruppe der vorliegenden Anmeldung ist also mit sich selbst stapelbar, d. h. zwei gemäß der obigen Aufgabenlösung ausgestaltete und daher gleichartige Umschaltventilbaugruppen können derart miteinander in Stapeleingriff gebracht und somit miteinander gestapelt werden, dass die Ausgabe-Anschlussöffnung der einen Umschaltventilbaugruppe in eine Quellen-Anschlussöffnung der jeweils anderen Umschaltventilbaugruppe liefern kann. Die in Lieferrichtung auf die Ausgabe-Anschlussöffnung folgende Quellen-Anschlussöffnung ist daher in der vorliegenden Anmeldung als "Folge-Anschlussöffnung" bezeichnet.

Die für diese Art von Stapelbarkeit erforderliche Ausgestaltung ist, ebenso wie bei stapelbaren Spielzeug-Bausteinen (etwa bei Lego^{®}-Steinen), ohne weiteres an einer einzelnen Umschaltventilbaugruppe feststellbar. Diese muss den ersten und den zweiten Stapel-Außenflächenabschnitt an unterschiedlichen Orten, also zueinander versetzt, aufweisen, sodass der erste Stapel-Außenflächenabschnitt dieser Umschaltventilbaugruppe mit dem zweiten Stapel-Außenflächenabschnitt einer gleichartigen Umschaltventilbaugruppe unter Bildung eines über beide Umschaltventilbaugruppen hinweg kontinuierlichen Gaslieferleitungsbereichs miteinander verbunden werden können, oder/und sodass der zweite Stapel-Außenflächenabschnitt dieser Umschaltventilgruppe mit dem ersten Stapel-Außenflächenabschnitt einer gleichartigen Umschaltventilbaugruppe unter Bildung eines über beide Umschaltventilbaugruppen hinweg kontinuierlichen Gaslieferleitungsbereichs miteinander verbunden werden können. Folglich sind der erste und der zweite Stapel-Außenflächenabschnitt ein und derselben Umschaltventilbaugruppe der vorliegenden Anmeldung grundsätzlich dazu ausgebildet, miteinander in den genannten Stapeleingriff gebracht zu werden, durch welchen der zur Ausgabe-Anschlussöffnung führende Gaslieferleitungsabschnitt mit dem von einer Quellen-Anschlussöffnung ausgehenden und in das Gehäuse führenden Gaslieferleitungsabschnitt zu einem gemeinsamen Gaslieferleitungsbereich verbunden werden.

Es kann also die Umschaltventilbaugruppe so in zwei Baugruppenteile geteilt gedacht werden, dass jeder Baugruppenteil einen anderen Außenflächenabschnitt der beiden Außenflächenabschnitte mit dem jeweils zugeordneten Stapel-Außenflächenabschnitt aufweist. Diese beiden Baugruppenteile sind dann durch Herstellung eines Stapeleingriffs der beiden Stapel-Außenflächenabschnitte der jeweiligen Baugruppenteile miteinander stapelbar.

Bevorzugt bilden die Gehäuse von gestapelten gleichartigen Umschaltventilbaugruppen wenigstens abschnittsweise eine gemeinsame bündige Außenfläche. Bevorzugt sind wenigstens 75 %, vorzugsweise wenigstens 80 %, der Länge einer von außen erkennbaren Trennfugenlinie einer zwischen zwei gestapelten Umschaltventilbaugruppen gebildeten Trennfuge in einer von den beiden Umschaltventilbaugruppen gebildeten gemeinsamen bündigen Außenfläche angeordnet.

Grundsätzlich reicht es zur Sicherstellung der gewünschten Stapelbarkeit aus, wenn ein von der Ausgabe-Anschlussöffnung ausgehender und in das Gehäuse hinein verlaufender Gaslieferleitungsabschnitt und ein von der Folge-Anschlussöffnung ausgehender und in das Gehäuse hineinverlaufender Gaslieferleitungsabschnitt bei hergestelltem Stapeleingriff miteinander einen kontinuierlichen Gaslieferleitungsbereich bilden. Bevorzugt sind die Ausgabe-Anschlussöffnung und die Folge-Anschlussöffnung derart am Gehäuse angeordnet, dass sie bei hergestelltem Stapeleingriff miteinander fluchten, was die Abdichtung der Verbindung von Ausgabe- und Folge-Anschlussöffnung gegen einen Gasdurchtritt zwischen dem Gaslieferleitungsbereich und der Außenumgebung erleichtert.

Zur Sicherstellung der Stapelbarkeit kann die Folge-Anschlussöffnung und die Ausgabe-Anschlussöffnung ein und derselben Umschaltventilbaugruppe von ein und dieselben Öffnungsachse durchsetzt sein, wobei die Öffnungsachse bevorzugt parallel zu einer Stapelachse verläuft, längs welcher eine Mehrzahl von gleichartigen modularen Umschaltventilbaugruppen stapelbar ist. Bevorzugt sind die Folge-Anschlussöffnung und die Ausgabe-Anschlussöffnung konzentrisch zur Öffnungsachse angeordnet. Ebenso sind aus Gründen möglichst einfacher Fertigung bevorzugt die jeweils von der Folge-Anschlussöffnung und der Ausgabe-Anschlussöffnung ausgehend in das Gehäuse hinein verlaufenden Gaslieferleitungsabschnitte von der Öffnungsachse durchsetzt, besonders bevorzugt zur Öffnungsachse konzentrisch.

Im Bereich einer Anschlussöffnung kann zur sicheren Verbindung mit einer Gasquelle bzw. mit einem Verbraucher eine Anschlussformation vorgesehen sein. Die Anschlussformation kann ein Gewinde, ein Teil eines Bajonettverschlusses oder ein männlicher oder weiblicher Teil einer an sich bekannten Schnellkupplung für Gasleitungen sein. Bevorzugt ist jede Anschlussöffnung mit einer Anschlussformation versehen. Damit eine Anschlussformation bei einer Handhabung der Umschaltventilbaugruppe nicht mit Strukturen außerhalb der Umschaltventilbaugruppe kollidiert und beschädigt wird, ist wenigstens eine Anschlussformation, sind vorzugsweise alle Anschlussformationen, einer Umschaltventilbaugruppe bevorzugt von einer Mündung oder/und einem Rand der der Anschlussformation zugeordneten Anschlussöffnung ins Gehäuse hinein zurückgesetzt angeordnet.

Dadurch, dass die Stapel-Außenflächenabschnitte an einem Außenseitenabschnitt des Gehäuses gelegen sind, ist sichergestellt, dass die komplementären Stapel-Außenflächenabschnitte gesonderter, aber gleichartiger Umschaltventilbaugruppen miteinander in Eingriff bringbar sind.

Grundsätzlich kann daran gedacht sein, die eine oder mehreren modularen Umschaltventilbaugruppen in einem dafür vorgesehenen Stapelrahmen anzuordnen, um insbesondere zwei oder mehr modulare Umschaltventilbaugruppen in einem Stapeleingriff miteinander zu halten. Ein solcher Stapelrahmen als zusätzliches Bauteil ist jedoch ohne Verlust einer Sicherbarkeit der Umschaltventilbaugruppen im Stapeleingriff entbehrlich, wenn die Umschaltventilbaugruppe Verbindungsmittel zur Verbindung mit einer gleichartigen modularen Umschaltventilbaugruppe aufweist. Beispielsweise kann die Umschaltbaugruppe einen Eingriffsvorsprung und eine Eingriffsausnehmung aufweisen, wobei der Eingriffsvorsprung, etwa ein beweglicher Eingriffshaken, zum Eingriff mit der Eingriffsausnehmung einer gleichartigen modularen Umschaltventilbaugruppe ausgebildet ist.

Eine einfache, aber effektive Möglichkeit, gleichartige modulare Umschaltventilbaugruppen in einem Stapeleingriff miteinander zu sichern, kann dadurch realisiert sein, dass an jedem Außenseitenabschnitt aus dem ersten und dem zweiten Außenseitenabschnitt je wenigstens eine Verbindungsformation als Verbindungsmittel vorgesehen ist, welche zur Sicherung einer körperlichen Verbindung der modularen Umschaltventilbaugruppe mit einer gleichartigen gesonderten modularen Umschaltventilbaugruppe ausgebildet ist. Dann können miteinander zu einer gestapelten Umschaltventilbaugruppenkaskade verbundene modulare Umschaltventilbaugruppen unmittelbar miteinander verbunden und aneinander im Stapeleingriff gesichert werden.

Die Stapelbarkeit einer modularen Umschaltventilbaugruppe mit einer gleichartigen modularen Umschaltventilbaugruppe kommt bei Ausbildung der oben genannten Verbindungsformationen konstruktiv dadurch zum Ausdruck, dass eine räumliche Relativanordnung zwischen der Folge-Anschlussanordnung und der Verbindungsformation in dem die Folge-Anschlussanordnung aufweisenden Außenseitenabschnitt die gleiche räumliche Relativanordnung ist, welche zwischen der Ausgabe-Anschlussöffnung und der Verbindungsformation in dem die Ausgabe-Anschlussöffnung aufweisenden Außenseitenabschnitt besteht. Da die nach Herstellung eines Stapeleingriffs aneinander anliegenden oder einander gegenüberliegenden Außenseitenabschnitte üblicherweise zueinander bezüglich einer zur Stapelachse orthogonalen Spiegelsymmetrieebene spiegelbildlich ausgebildet sein müssen, können die räumlichen Relativanordnungen von Verbindungsformation und Folge-Anschlussanordnung im einen Außenseitenabschnitt einerseits sowie von Verbindungsformation und Ausgabe-Anschlussöffnung im jeweils anderen Außenseitenabschnitt andererseits an einer Umschaltventilbaugruppe spiegelbildlich vorliegen. Dies ändert jedoch nichts daran, dass die räumlichen Relativanordnungen unter Berücksichtigung der Spiegelsymmetrie dieselben sind, also die wenigstens eine Verbindungsformation und die Anschlussöffnung in jedem der beiden sie aufnehmenden Außenseitenabschnitte in identischen Richtungen und mit identischen Abständen voneinander angeordnet sind.

Die wenigstens eine Verbindungsformation des ersten Außenseitenabschnitt kann mit der wenigstens einen Verbindungsformation des zweiten Außenseitenabschnitts komplementär ausgebildet sein, beispielsweise als Vorsprung und Ausnehmung, wobei die beiden Verbindungsformationen aneinander festlegbar sind, beispielsweise durch Gewinde, durch einen Bajonettverschluss oder durch Kombination eines Rastvorsprungs mit einer Rastausnehmung. Eine Verbindungsformation im Sinne der vorliegenden Anmeldung ist auch eine das Gehäuse vom ersten zum zweiten Außenseitenabschnitt durchsetzende Durchgangsöffnung, durch welche ein Verbindungsmittel, wie etwa eine Gewindestange, eine Schraube oder eine Spannvorrichtung und dergleichen, hindurchführbar ist. Die Durchgangsöffnungen von längs einer Stapelachse gestapelten Umschaltventilbaugruppen fluchten vorzugsweise, sodass sie durch ein gemeinsames Verbindungsmittel durchsetzt und die Umschaltventilbaugruppen durch ein gemeinsames Verbindungsmittel pro Durchgangsöffnung aneinander im gestapelten Zustand sicherbar sind.

Um gewährleisten zu können, dass an eine modulare Umschaltventilbaugruppe, welche Teil eines Pakets von gestapelten modularen Umschaltventilbaugruppen ist, eine Atemgasquelle anschließbar ist, ist bevorzugt vorgesehen, dass die jeweils andere Quellen-Anschlussöffnung aus der ersten und der zweiten Quellen-Anschlussöffnung, welche nicht die Folge-Anschlussöffnung ist, in einem anderen Außenseitenabschnitt des Gehäuses als dem ersten und dem zweiten Außenseitenabschnitt vorgesehen ist. Der andere Außenseitenabschnitt liegt selbst dann noch frei, wenn der die Folge-Anschlussöffnung aufweisende Außenseitenabschnitt und der die Ausgabe-Anschlussöffnung aufweisende Außenseitenabschnitt sich in einem Stapel von gleichartigen modularen Umschaltventilbaugruppen befinden und von Außenseitenabschnitten anderer gleichartiger modularer Umschaltventilbaugruppen bedeckt sind.

Eine einfache und effektive Stapelung von zwei oder mehr gleichartigen modularen Umschaltventilbaugruppen bei gleichzeitig möglichst geringem erforderlichen Bauraumvolumen für den so erzeugten Stapel kann dadurch ermöglicht werden, dass der erste und der zweite Außenseitenabschnitt auf voneinander weg weisenden, entgegengesetzten Gehäuseseiten ausgebildet sind.

Die jeweils andere Quellen-Anschlussöffnung kann in einem dritten Außenseitenabschnitt ausgebildet sein, welcher den ersten und den zweiten Außenseitenabschnitt miteinander verbindet. Beispielsweise kann, dass das Gehäuse eine raumsparende parallelepiped-artige Außengestalt aufweisen. Dabei ist insbesondere an eine würfel- oder quaderförmige Gehäusegestalt gedacht, sodass mehrere gleichartige modulare Umschaltventilbaugruppen bei unveränderter Grundrissfläche durch bloßes Aufeinanderstapeln längs einer Stapelachse miteinander zu einem Stapel in Stapeleingriff bringen lassen. Wenngleich dies nicht zwingend notwendig ist, bildet an einem bevorzugten quaderförmigen Gehäuse der erste und der zweite Außenseitenabschnitt je eine andere Stirnseite des Gehäuses. Der dritte Außenseitenabschnitt kann dann an einer der seitlichen Mantelflächenabschnitte ausgebildet sein. Die Stirnseiten sind dabei bevorzugt die größten Seitenflächen des quaderförmigen Gehäuses, sodass ausreichend Platz sowohl für die Anordnung der jeweiligen Anschlussöffnung als auch für die Anordnung der oben bevorzugt genannten wenigstens einen Verbindungsformation zur Verfügung steht.

Bevorzugt sind bei einem würfel- oder quaderförmigen Gehäuse die Stapelachse und die zuvor genannte Öffnungsachse orthogonal zu zwei gegenüberliegenden, parallelen Außenseitenabschnitten orientiert.

Selbstverständlich kann an die Folge-Anschlussöffnung, eine herkömmliche Atemgasquelle, etwa eine Gasflasche, angeschlossen sein, falls die modulare Umschaltventilbaugruppe als einzige Umschaltventilbaugruppe zur Belieferung einer Beatmungsvorrichtung mit Atemgas verwendet wird.

Durch die komplementären Ausgestaltungen des ersten und des zweiten Stapel-Außenflächenabschnitts ist gewährleistet, dass der erste und der zweite Stapel-Außenflächenabschnitt zur Herstellung des Stapeleingriffs zusammenpassen. Der Stapeleingriff kann ein Formschlusseingriff von erstem und zweitem Stapel-Außenflächenabschnitt sein oder/und kann ein Anlageeingriff von erstem und zweitem Stapel-Außenflächenabschnitt sein.

Dann, wenn zwischen dem ersten und dem zweiten Stapel-Außenflächenabschnitt ein Formschlusseingriff als Stapeleingriff herstellbar sein soll, kann der erste Stapel-Außenflächenabschnitt eine Formation aus einem Vorsprung und einer Ausnehmung aufweisen und kann der zweite Stapel-Außenflächenabschnitt die jeweils andere Formation aus einem Vorsprung und einer Ausnehmung komplementär zur Formation des ersten Stapel-Außenflächenabschnitts aufweisen.

Im Falle eines Anlageeingriffs als dem Stapeleingriff können der erste Stapel-Außenflächenabschnitt und der zweite Stapel-Außenflächenabschnitt jeweils ebene oder komplementär gewölbte Stapel-Außenflächenabschnitte sein. Falls die modulare Umschaltventilbaugruppe gemäß einer bevorzugten Weiterbildung je wenigstens eine Verbindungsformation in dem ersten und dem zweiten Außenseitenabschnitt aufweist, genügen zwei Verbindungsformationen jeweils im ersten und im zweiten Außenseitenabschnitt für die korrekte relative Orientierung zu stapelnder modularer Umschaltventilbaugruppen miteinander.

Ebenso können der erste und der zweite Außenseitenabschnitt ebene Außenseitenabschnitte sein, wie das bei dem oben bevorzugt genannten parallelepiped-förmigen Gehäuse der Fall ist. Bevorzugt sind dann die Stapelachse und die zuvor genannte Öffnungsachse orthogonal zum ersten und zum zweiten Außenseitenabschnitt orientiert.

Zur Herstellung eines möglichst gasdichten kontinuierlichen Gaslieferleitungsbereichs ist an der Mündung bzw. am Rand wenigstens einer Anschlussöffnung aus Folge- und Ausgabe-Anschlussöffnung eine Dichtungsanordnung, etwa ein Dichtungsring, vorgesehen, welcher bei Herstellung eines Stapeleingriffs mit einer weiteren modularen gleichartigen Umschaltventilbaugruppe in Anlage an eine Struktur der weiteren Umschaltventilbaugruppe gelangt. Vorzugsweise ist an sowohl der Folgeals auch an der Ausgabe-Anschlussöffnung je eine Dichtungsanordnung angeordnet. Bevorzugt ist dann die Struktur der weiteren Umschaltventilbaugruppe, an welche die Dichtungsanordnung in Anlage gelangt, die Dichtungsanordnung an der weiteren Umschaltventilbaugruppe. Zur Erzielung einer möglichst guten Dichtungswirkung können die nach außen weisenden Flächen der Dichtungsanordnung an der Ausgabe-Anschlussöffnung einerseits und die Dichtungsanordnung an der Folge-Anschlussöffnung andererseits komplementär zueinander ausgebildet sein, so dass sie in der Art eines Schlüssel-Schloss-Prinzips ineinander passen und bei Anlage aneinander zusätzlich oder alternativ zu einer Dichtungswirkung durch verformende Anlage aneinander eine Art Labyrinthdichtung bilden können.

Grundsätzlich können die Stapel-Außenflächenabschnitte beliebig ausgestaltet sein. Bevorzugt weisen Sie jedoch die ihnen räumlich jeweils zugeordnete Anschlussöffnung auf, sodass die zuvor genannte Dichtungsanordnung bevorzugt im jeweiligen Stapel-Außenflächenabschnitt angeordnet ist. Es ist jedoch auch möglich, die Stapel-Außenflächenabschnitte nur zur Sicherstellung einer korrekten Relativanordnung zwischen zwei gleichartigen modularen Umschaltventilbaugruppen zu nutzen. Die korrekte Relativanordnung stellt dann sicher, dass bei hergestelltem Stapeleingriff die mit Abstand von den komplementären Stapel-Außenflächenabschnitten in den jeweiligen Außenseitenabschnitten angeordneten Anschlussöffnungen und ihre von den jeweiligen Anschlussöffnungen ausgehend ins Gehäuse hinein verlaufenden Gaslieferleitungsabschnitte miteinander zu einem die Anschlussöffnungen überschreitenden kontinuierlichen Gaslieferleitungsbereich verbunden sind.

Eine Umschaltventilanordnung der eingangs genannten Art, insbesondere die vorstehend beschriebene modulare stapelbare Umschaltventilanordnung, kann bei Verlagerung eines Ventilkörpers seiner Ventilanordnung zwischen seinen beiden den unterschiedlichen genannten Betriebszuständen der Ventilanordnung zugeordneten Betriebsstellungen im Bereich der für einen Durchfluss zu sperrenden Gaslieferleitung unerwünschterweise ein Gasvolumen einschließen. Das eingeschlossene Gasvolumen kann dann wie eine Gasfeder der eigentlich gewünschten Verstellung des Ventilkörpers in die gewünschte Betriebsstellung entgegenwirken und im nachteiligsten Fall das Erreichen der Betriebsstellung verhindern oder nur ein kurzes Erreichen der Betriebsstellung ermöglichen und anschließend den Ventilkörper wieder aus der eigentlich gewünschten Betriebsstellung entfernen.

Die aus der DE 29 18 791 A1 bekannte Umschaltventilbaugruppe weist zwei gesonderte Ventilkörper in Gestalt von translatorisch verlagerbaren Ventilkolben auf, von welchen der der Ausgabe-Anschlussöffnung näher gelegene Ventilkolben in jenem Bereich, in welchem er eine Gaslieferleitung sperrt, über einen mechanischen Stößel ein Ventil öffnet, durch welches die gesperrte Gaslieferleitung mit druckgemindertem Gas gespült wird, das stromabwärts der Ausgabe-Anschlussöffnung aus einer an die Ausgabe-Anschlussöffnung angeschlossenen Gasleitung stromabwärts eines Druckminderers abgeleitet wird. Auch in der aus der DE 29 18 791 A1 bekannten Umschaltventilbaugruppe ist aufgrund ihrer Konstruktion das Einschließen eines Gasvolumens im Bereich der zu sperrenden Gaslieferleitung mit den oben genannten nachteiligen Wirkungen möglich.

Zusätzlich besteht an der bekannten Umschaltventilbaugruppe dann, wenn die zunächst gesperrte Gaslieferleitung wieder für einen Durchfluss von Gas freigegeben wird, für eine kurze, aber durchaus relevante Zeitdauer ein unmittelbarer Strömungspfad von der unter sehr hohem Druck stehenden Gasquelle in die zum Verbraucher führende Gasleitung stromabwärts des Druckminderers. Ein solcher selbst nur kurzzeitig bestehender Strömungspfad kann im Falle einer Beatmung eines Patienten fatale Wirkungen entfalten.

Angesichts der vorstehend geschilderten Nachteile weist die Ventilanordnung einer Umschaltventilbaugruppe der eingangs genannten Art, welche bevorzugt zur Versorgung von Patienten mit Atemgas beiträgt, insbesondere einer oben beschriebenen modularen stapelbaren Umschaltventilbaugruppe, einen ersten und einen vom ersten verschiedenen zweiten Ventilkörper auf, wobei der erste und der zweite Ventilkörper jeweils im Strömungspfad zwischen der ersten und der zweiten Quellen-Anschlussöffnung einerseits und der Ausgabe-Anschlussöffnung andererseits angeordnet sind, wobei der erste Ventilkörper in einer ersten Ventilkörperkavität verlagerbar aufgenommen ist und wobei der zweite Ventilkörper in einer mit Abstand von der ersten Ventilkörperkavität angeordneten zweiten Ventilkörperkavität verlagerbar aufgenommen ist, wobei der erste und der zweite Ventilkörper jeweils verlagerbar ist zwischen einer dem ersten Betriebszustand zugeordneten ersten Betriebsstellung und einer dem zweiten Betriebszustand zugeordneten zweiten Betriebsstellung, wobei die Umschaltventilbaugruppe eine zwischen dem ersten Ventilkörper und der ersten Ventilkörperkavität wirksame erste Dichtungsanordnung aufweist, welche in der ersten Ventilkörperkavität einen ersten Kavitätsbereich und einen vom ersten verschiedenen zweiten Kavitätsbereich definiert, wobei die Umschaltventilbaugruppe eine zwischen dem zweiten Ventilkörper und der zweiten Ventilkörperkavität wirksame zweite Dichtungsanordnung aufweist, welche in der zweiten Ventilkörperkavität einen dritten Kavitätsbereich und einen vom dritten verschiedenen vierten Kavitätsbereich definiert, wobei ein Kavitätsbereich aus erstem und drittem Kavitätsbereich als erster Liefer-Kavitätsbereich Teil der ersten Gaslieferleitung, aber nicht Teil der zweiten Gaslieferleitung ist, und wobei ein Kavitätsbereich aus zweitem und viertem Kavitätsbereich als zweiter Liefer-Kavitätsbereich Teil der zweiten Gaslieferleitung, aber nicht Teil der ersten Gaslieferleitung ist,
wobei die Umschaltventilbaugruppe eine erste Entlüftungsleitung aufweist, welche den ersten Liefer-Kavitätsbereich mit einer von der Ausgabe-Anschlussöffnung verschiedenen ersten Senken-Anschlussöffnung verbindet,
wobei die Umschaltventilbaugruppe eine zweite Entlüftungsleitung aufweist, welche den zweiten Liefer-Kavitätsbereich mit einer von der Ausgabe-Anschlussöffnung verschiedenen zweiten Senken-Anschlussöffnung verbindet,
wobei jener andere Kavitätsbereich aus erstem und drittem Kavitätsbereich, welcher nicht erster Liefer-Kavitätsbereich ist, als erster Entlüftungs-Kavitätsbereich Teil der ersten Entlüftungsleitung, aber nicht der ersten Gaslieferleitung ist, und wobei jener andere Kavitätsbereich aus zweitem und viertem Kavitätsbereich, welcher nicht zweiter Liefer-Kavitätsbereich ist, als zweiter Entlüftungs-Kavitätsbereich Teil der zweiten Entlüftungsleitung, aber nicht der zweiten Gaslieferleitung ist.

Durch die Ausbildung der ersten und der zweiten Entlüftungsleitung, welche jeweils zu einer Senken-Anschlussöffnung führen, kann Gas aus der Ventilkörperkavität zu einer Gassenke abgeleitet werden. Damit kann ein Einschluss einer Gasmenge durch den in der Ventilkörperkavität aufgenommenen Ventilkörper in einem kritischen Kavitätsbereich verhindert werden. Der kritische Kavitätsbereich muss hierzu lediglich Teil der ersten oder der zweiten Entlüftungsleitung sein. Da zwei Betriebsstellungen von Ventilkörpern bestehen, in welchen jeweils eine andere Gaslieferleitung gesperrt ist, können auch zwei kritische Kavitätsbereiche existieren, in welchen der Einschluss einer Gasmenge durch den Ventilkörper dem Erreichen der sperrenden Betriebsstellung oder/und dem Verbleib in dieser entgegenwirkt. Vorteilhaft kann zur Entlüftung der beiden kritischen Kavitätsbereiche jeder dieser beiden kritischen Kavitätsbereiche Teil einer anderen Entlüftungsleitung aus der ersten oder der zweiten Entlüftungsleitung sein.

Im einfachsten und daher bevorzugten Fall ist die Gassenke die Umgebungsatmosphäre, sodass die Senken-Anschlussöffnung lediglich eine Öffnung der jeweiligen Entlüftungsleitung in die Umgebungsatmosphäre sein kann.

Es soll jedoch nicht ausgeschlossen sein, dass an wenigstens einer Senken-Anschlussöffnung ein Filter oder/und ein Gasauffangbehälter angeschlossen ist.

Die Gassenke der ersten Entlüftungsleitung kann von der Gassenke der zweiten Entlüftungsleitung verschieden sein. Für eine bevorzugte Beatmungsanwendung reicht es jedoch in der Regel aus, wenn beide Gassenken dieselbe Gassenke sind. Die erste und die zweite Senken-Anschlussöffnung können dann gemeinsam in einer einzigen Senken-Anschlussöffnung realisiert sein. Die erste und die zweite Entlüftungsleitung können dann zumindest in einem der gemeinsamen Senken-Anschlussöffnung nahen Bereich einen gemeinsamen Entlüftungsleitungsabschnitt aufweisen. Aus Gründen einer einfacheren Fertigung kann jedoch die gesonderte Ausbildung der ersten und der zweiten Senken-Anschlussöffnung sowie der ersten und der zweiten Entlüftungsleitung bevorzugt sein. Dies gilt insbesondere dann, wenn die erste und die zweite Entlüftungsleitung als gebohrte Entlüftungsleitungen ausgebildet sind und zu Entlüftungs-Kavitätsbereichen führen, welche räumlich mit einer Abstandskoordinate voneinander entfernt gelegen sind, die quer zu den jeweiligen Bohrungsachsen der Entlüftungsleitungen verläuft.

Grundsätzlich kann jeder der beiden Ventilkörper derart ausgestaltet sein, dass er auf einer Wirkseite eine Gaslieferleitung sperrt oder freigibt und auf seiner anderen Wirkseite eine Gasströmung in einer Entlüftungsleitung beeinflusst. Allerdings ist ein solcher Aufbau kompliziert und erfordert die Einhaltung bestimmter Randbedingungen an den Ventilkörpern. Eine eindeutigere Funktionszuordnung und eine größere konstruktive Freiheit kann dann vorteilhaft ausgenutzt werden, wenn der erste Kavitätsbereich der erste Liefer-Kavitätsbereich ist, der zweite Kavitätsbereich der zweite Liefer-Kavitätsbereich ist, der dritte Kavitätsbereich der erste Entlüftungs-Kavitätsbereich ist und der vierte Kavitätsbereich der zweite Entlüftungs-Kavitätsbereich ist. Dann sind beide Liefer-Kavitätsbereiche durch die eine der beiden Ventilkörperkavitäten realisiert und sind beide Entlüftungs-Kavitätsbereiche durch die jeweils andere der beiden Ventilkörperkavitäten realisiert. In der Folge ist dann beiden Liefer-Kavitätsbereichen der eine der beiden Ventilkörper zugeordnet und ist beiden Entlüftungs-Kavitätsbereichen der jeweils andere Ventilkörper zugeordnet. So kann jede Ventilkörperkavität und jeder darin aufgenommenen Ventilkörper für die ihm zugedachte Ventil-Aufgabe ausgelegt und realisiert werden.

Zur Realisierung der Ventilanordnung und ihrer Entlüftungsfunktion kann eine den ersten Entlüftungs-Kavitätsbereich umgebende Wand, insbesondere Kavitätswand, einen ersten Entlüftungs-Ventilkörperbereich eines Ventilkörpers umgeben und kann eine den zweites Entlüftungs-Kavitätsbereich umgebende Wand, insbesondere Kavitätswand, einen zweiten Entlüftungs-Ventilkörperbereich eines Ventilkörpers umgeben, wobei der erste Entlüftungs-Ventilkörperbereich dazu ausgebildet ist, abhängig von der Betriebsstellung des den ersten Entlüftungs-Ventilkörperbereich aufweisenden Ventilköpers, die erste Entlüftungsleitung für eine Gasströmung zu öffnen oder zu schließen, und wobei der zweite Entlüftungs-Ventilkörperbereich dazu ausgebildet ist, abhängig von der Betriebsstellung des den zweiten Entlüftungs-Ventilkörperbereich aufweisenden Ventilköpers, die zweite Entlüftungsleitung für eine Gasströmung zu öffnen oder zu schließen. Bevorzugt sind, wie oben bereits dargelegt, der erste und der zweite Entlüftungs-Ventilkörperbereich am selben Ventilkörper ausgebildet.

Ebenso kann zur Sicherstellung der Lieferfunktion der Ventilanordnung eine den ersten Liefer-Kavitätsbereich umgebende Wand, insbesondere Kavitätswand, einen ersten Liefer-Ventilkörperbereich umgeben und kann eine den zweiten Liefer-Kavitätsbereich umgebende Wand, insbesondere Kavitätswand, einen zweiten Liefer-Ventilkörperbereich umgeben, wobei der erste Liefer-Ventilkörperbereich dazu ausgebildet ist, die erste Gaslieferleitung abhängig von der Betriebsstellung des den ersten Liefer-Ventilkörperbereich aufweisenden Ventilkörpers für eine Gasströmung zu sperren oder freizugeben, und wobei der zweite Liefer-Ventilkörperbereich dazu ausgebildet ist, die zweite Gaslieferleitung abhängig von der Betriebsstellung des den zweiten Liefer-Ventilkörperbereich aufweisenden Ventilkörpers für eine Gasströmung zu sperren oder freizugeben. Bevorzugt sind, wie oben bereits dargelegt, der erste und der zweite Liefer-Ventilkörperbereich am selben Ventilkörper ausgebildet. Dies ist bevorzugt ein Ventilkörper, an welchem kein Entlüftungs-Ventilkörperbereich ausgebildet ist.

Grundsätzlich kann daran gedacht sein, dass sowohl der erste als auch der zweite Ventilkörper in ihrer ersten Betriebsstellung beide die zweite Entlüftungsleitung sperren und in ihrer zweiten Betriebsstellung beide die erste Entlüftungsleitung sperren. Da mit der Entlüftung jedoch nur eine Restgasmenge aus einer Ventilkörperkavität ausgelassen werden soll, ist eine doppelte Schließung der Entlüftungsleitungen durch zwei bzw. durch beide Ventilkörper gleichzeitig unnötig. Bevorzugt ist daher vorgesehen, dass von dem ersten und dem zweiten Ventilkörper nur der den ersten Entlüftungs-Ventilkörperbereich aufweisende Ventilkörper dazu ausgebildet ist, die erste Entlüftungsleitung für eine Gasströmung zu öffnen oder zu schließen, oder/und dass von dem ersten und dem zweiten Ventilkörper nur der den zweiten Entlüftungs-Ventilkörperbereich aufweisende Ventilkörper dazu ausgebildet ist, die zweite Entlüftungsleitung für eine Gasströmung zu öffnen oder zu schließen.

Grundsätzlich kann daran gedacht sein, dass sowohl der erste als auch der zweite Ventilkörper in ihrer ersten Betriebsstellung beide die erste Gaslieferleitung sperren und in ihrer zweiten Betriebsstellung beide die zweite Gaslieferleitung sperren. Gerade durch die vorteilhafte Entlüftung, die die Ventilanordnung der vorliegend diskutierten Umschaltventilbaugruppe ermöglicht, ist eine doppelte Sperrung der Gaslieferleitungen durch zwei Ventilkörper unnötig. Bevorzugt ist daher vorgesehen, dass von dem ersten und dem zweiten Ventilkörper nur der den ersten Liefer-Ventilkörperbereich aufweisende Ventilkörper dazu ausgebildet ist, die erste Gaslieferleitung für eine Gasströmung zu sperren oder freizugeben, oder/und dass von dem ersten und dem zweiten Ventilkörper nur der den zweiten Liefer-Ventilkörperbereich aufweisende Ventilkörper dazu ausgebildet ist, die erste Gaslieferleitung für eine Gasströmung zu sperren oder freizugeben.

Für eine besonders bauraumsparende Ausgestaltung kann im ersten Liefer-Ventilkörperbereich eine erste Gasleitung ausgebildet sein, welche Teil der ersten Gaslieferleitung ist und kann im zweiten Liefer-Ventilkörperbereich eine zweite Gasleitung ausgebildet sein, welche Teil der zweiten Gaslieferleitung ist. Zusätzlich oder alternativ kann im ersten Liefer-Ventilkörperbereich eine erste Gasleitung ausgebildet sein, welche Teil der ersten Entlüftungsleitung ist und kann im zweiten Liefer-Ventilkörperbereich eine zweite Gasleitung ausgebildet sein, welche Teil der zweiten Entlüftungsleitung ist. Um genau jenen kritischen Kavitätsbereich entlüften zu können, in welchem eine eingeschlossene Gasmenge eine besonders nachteilige Wirkung auf die Positionierung des Ventilkörpers ausüben kann, ist bevorzugt die im ersten Liefer-Ventilkörperbereich ausgebildete erste Gasleitung Teil sowohl der ersten Gaslieferleitung als auch Teil der ersten Entlüftungsleitung. Aus demselben Grund ist bevorzugt die im zweiten Liefer-Ventilkörperbereich ausgebildete zweite Gasleitung Teil sowohl der zweiten Gaslieferleitung als auch der zweiten Entlüftungsleitung.

Bevorzugt sind sowohl der erste als auch der zweite Ventilkörper nur durch die in der Umschaltventilbaugruppe herrschenden Gasdrücke zwischen ihrer ersten und ihrer zweiten Betriebsstellung verlagerbar. Dadurch können der erste Ventilkörper und der zweite Ventilkörper und auch das die beiden Ventilkörper aufnehmende Gehäuse frei von auf den ersten und den zweiten Ventilkörper wirkenden mechanischen Vorspannmitteln sein. Dies reduziert die Bauteilanzahl der Umschaltventilbaugruppe und damit die Anzahl möglicher Fehlerquellen und die Anzahl möglicher Wartungserfordernisse.

Als Umschaltventilbaugruppe für eine Beatmungsvorrichtung ist die vorliegend diskutierte Umschaltventilbaugruppe bevorzugt auch schmiermittelfrei, sodass kein Schmierstoff zu Atemwegen eines Patienten transportiert werden kann.

Wenigstens einer der beiden Ventilkörper kann ein rotatorisch um eine Ventilkörper-Schwenkachse zwischen seinen Betriebsstellungen verlagerbarer Ventilkörper sein. Bevorzugt ist wenigstens einer der beiden Ventilkörper, besonders bevorzugt sind beide Ventilkörper translatorisch zwischen ihren Betriebsstellungen verlagerbare Ventilkörper, höchstbevorzugt Ventilkolben. Zur Verlagerung zwischen ihren Betriebsstellungen nur durch Gasdrücke in den Gaslieferleitungen ist wenigstens ein Ventilkörper, sind vorzugsweise beide Ventilkörper, bevorzugt ein doppeltwirkender Ventilkolben. Bevorzugt ist jeder Ventilkörper ein doppeltwirkender Ventilkolben, dessen eine Kolbenwirkfläche mit Gas aus der ersten Gaslieferleitung und dessen entgegengesetzt wirkende andere Kolbenwirkfläche mit Gas aus der zweiten Gaslieferleitung beaufschlagt ist.

Die vorliegende Erfindung betrifft außerdem eine Umschaltventilanordnung, umfassend eine erste Umschaltventilbaugruppe und eine zweite Umschaltventilbaugruppe, jeweils ausgestaltet gemäß obiger Beschreibung, wobei der erste Stapel-Außenflächenabschnitt der einen Umschaltventilbaugruppe aus erster und zweiter Umschaltventilbaugruppe mit dem zweiten Stapel-Außenflächenabschnitt der jeweils anderen Umschaltventilbaugruppe aus erster und zweiter Umschaltventilbaugruppe unter Bildung eines über die Auslass-Anschlussanordnung der einen Umschaltventilbaugruppe und die Folge-Anschlussanordnung der anderen Umschaltventilbaugruppe hinweg kontinuierlichen Gaslieferleitungsbereichs in Stapeleingriff miteinander stehen.

Bevorzugt umfasst die Umschaltventilanordnung eine an sich bekannte Priorisierungsventilbaugruppe mit einem Gehäuse, mit einer am Gehäuse ausgebildeten ersten Quellen-Anschlussöffnung zum Anschluss einer ersten Gasquelle, mit einer am Gehäuse ausgebildeten zweiten, gegenüber der ersten priorisierten Quellen-Anschlussöffnung zum Anschluss einer zweiten Gasquelle, und mit einer am Gehäuse ausgebildeten Ausgabe-Anschlussöffnung zum Anschluss eines Verbrauchers, um diesen mit Gas aus der ersten oder der zweiten Gasquelle zu versorgen. Die Priorisierungsventilbaugruppe umfasst eine erste Gaszufuhrleitung, welche die erste Quellen-Anschlussöffnung mit der Abgabe-Anschlussöffnung verbindet, und eine zweite Gaszufuhrleitung, welche die zweite Quellen-Anschlussöffnung mit der Ausgabe-Anschlussöffnung der Priorisierungsventilbaugruppe verbindet. Die Priorisierungsventilbaugruppe umfasst weiter eine Priorisierungsventilanordnung, deren doppeltwirkender Ventilkörper derart unterschiedlich bemessene, in entgegengesetzten Richtungen wirkende Wirkflächen aufweist, dass er die erste Gaszufuhrleitung sperrt und die zweite Gaszufuhrleitung öffnet, wenn an der zweiten Quellen-Anschlussöffnung eine Gasquelle mit einem vorbestimmten Mindest-Gasdruck angeschlossen ist, und zwar unabhängig davon, ob an der ersten Quellen-Anschlussöffnung eine vorbestimmte Gasquelle angeschlossen ist oder nicht und unabhängig vom Füllgrad der vorbestimmten Gasquelle. Mit einer solchen Priorisierungsventilanordnung kann einer klinischen gebäudeinstallierten Atemgasquelle als der zweiten Gasquelle stets Durchleitungsvorrang zur Ausgabe-Anschlussöffnung der Priorisierungsventilbaugruppe vor einer Atemgasflasche als der ersten Gasquelle eingeräumt werden.

Bevorzugt ist die Priorisierungsventilanordnung mit einer oben beschriebenen Umschaltventilanordnung derart stapelbar, dass die erste Quellen-Anschlussöffnung der Priorisierungsventilanordnung und die ausgehend von ihr ins Gehäuse der Priorisierungsventilanordnung verlaufende erste Gaszufuhrleitung in einem mit der Umschaltventilanordnung gestapelten Zustand an die Ausgabe-Anschlussöffnung der Umschaltventilanordnung anschließen.

Bevorzugt ist das Gehäuse der Priorisierungsventilanordnung wenigstens in einer Ebene orthogonal zur Stapelachse mit gleichen Abmessungen wie das Gehäuse der Umschaltventilanordnung ausgestaltet, sodass die Priorisierungsventilanordnung und die Umschaltventilanordnung gemeinsam eine im Wesentlichen bündige Außenfläche bilden können. Die Priorisierungsventilanordnung kann zur dauerhaften Verbindung mit der Umschaltventilanordnung wenigstens eine Verbindungsformation aufweisen, welche zu der wenigstens einen Verbindungsformation der Umschaltventilanordnung passt. Für die Verbindungsformation der Priorisierungsventilanordnung gilt daher das oben zur Verbindungsformation der Umschaltventilanordnung Gesagte entsprechend.

Die Priorisierungsventilanordnung kann zusätzlich oder alternativ zur oben genannten zweiten Umschaltventilbaugruppe in der Umschaltventilanordnung vorgesehen sein.

Weiter betrifft die vorliegende Erfindung eine Beatmungsvorrichtung, vorzugsweise mobile Beatmungsvorrichtung zur Versorgung eines Patienten mit Atemgas, umfassend einen Atemgaseingang, einen Atemgasausgang, eine Atemgasleitung, welche den Atemgaseingang mit dem Atemgasausgang verbindet, eine Strömungsveränderungsvorrichtung zur Veränderung der pro Zeiteinheit durch den Atemgasausgang strömenden Atemgasmenge oder/und zur Veränderung des Atemgasdrucks am Atemgasausgang und eine Steuereinrichtung zur Steuerung des Betriebs der Strömungsveränderungsvorrichtung, wobei stromaufwärts des Atemgaseingangs die Ausgabe-Anschlussöffnung wenigstens einer gemäß obiger Beschreibung ausgestalteten Umschaltventilbaugruppe oder einer gemäß obiger Beschreibung ausgestalteten Umschaltventilanordnung mit dem Atemgaseingang der Beatmungsvorrichtung gasübertragend verbunden ist.

Die vorliegende Erfindung wird nachfolgend anhand der beiliegenden Zeichnungen näher erläutert werden. Es stellt dar:
- Fig. 1: eine perspektivische Ansicht des Gehäuses einer erfindungsgemäßen Umschaltventilbaugruppe mit Blick auf die Ausgabe-Anschlussöffnung,
- Fig. 2: eine Längsschnittansicht durch das Gehäuse entlang der zu den beiden Stirnseiten parallelen Schnittebene II-II in Figur 3 und Figur 4,
- Fig. 3: eine Längsschnittansicht durch das Gehäuse entlang der zu den Stirnseiten orthogonalen und zu den Längsabschnitten der Mantelfläche parallelen Schnittebene III-III von Figur 2,
- Fig. 4: eine Draufsicht auf den in Figur 1 vom Betrachter abgewandten Längsabschnitt der Mantelfläche, bei Betrachtung des Gehäuses längs des Pfeils IV von Figur 2,
- Fig. 5: eine Querschnittsansicht entlang der zu den Stirnseiten orthogonalen und zu den Querabschnitten der Mantelfläche parallelen Schnittebene V-V von Figur 2,
- Fig. 6: eine Draufsicht auf den in Figur 1 vom Betrachter abgewandten Querabschnitt der Mantelfläche, bei Betrachtung des Gehäuses entlang des Pfeils VI von Figur 2,
- Fig. 7: Draufsicht auf die in Figur 1 vom Betrachter abgewandte Stirnseite des Gehäuses mit einer Quellen-Anschlussöffnung, welche Folge-Anschlussöffnung des Gehäuses ist,
- Fig. 8: eine Längsschnittansicht durch die erfindungsgemäße Umschaltventilbaugruppe mit der Ventilanordnung im ersten Betriebszustand, wobei die Schnittebene durch die Umschaltventilbaugruppe dieselbe Schnittebene wie jene der Figur 2 ist,
- Fig. 9: eine Längsschnittansicht gemäß Figur 8 durch die erfindungsgemäße Umschaltventilbaugruppe mit der Ventilanordnung in einem Zwischen-Betriebszustand zwischen dem ersten Betriebszustand von Figur 8 und dem zweiten Betriebszustand von Figur 10,
- Fig. 10: eine Längsschnittansicht gemäß Figur 8 durch die erfindungsgemäße Umschaltventilbaugruppe mit der Ventilanordnung im zweiten Betriebszustand,
- Fig. 11: eine Längsschnittansicht gemäß Figur 8 durch die erfindungsgemäße Umschaltventilbaugruppe mit der Ventilanordnung in einem Zwischen-Betriebszustand zwischen dem zweiten Betriebszustand von Figur 10 und dem ersten Betriebszustand von Figur 8,
- Fig. 12: eine perspektivische Ansicht eines Stapels aus Umschaltventilanordnungen der Fig. 1 bis 11 und einer Priorisierungsventilbaugruppe in einem Beatmungssystem zur unterbrechungsfreien Versorgung eines Patienten mit Atemgas aus mehreren nacheinander liefernden Atemgasquellen,
- Fig. 13: eine perspektivische Explosionszeichnung des Stapels von Fig. 12, und
- Fig. 14: eine Längsschnittansicht durch Folge- und Ausgabe-Anschlussöffnungen am Stapel von Fig. 12 und 13 sowie durch die von diesen ausgehenden Gaslieferleitungsabschnitte, die einen gemeinsamen, kontinuierlichen Gaslieferleitungsbereich bilden.

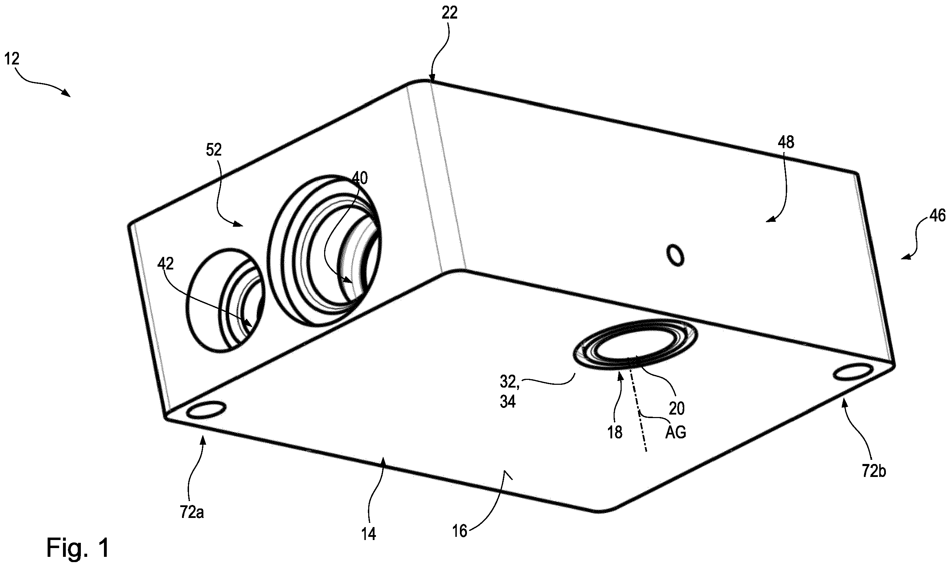

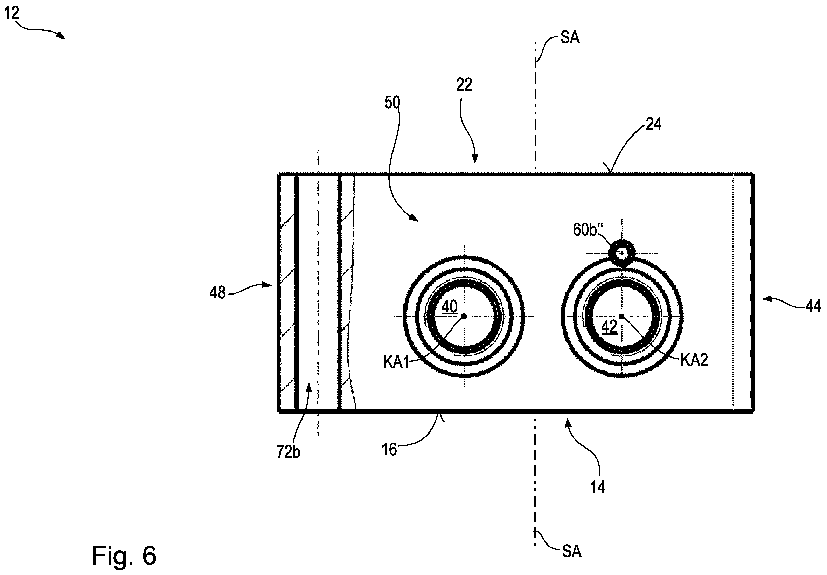

Anhand der gemeinsam zu betrachtenden Figuren 1 bis 7 wird zunächst die komplexe Leitungsstruktur innerhalb des Gehäuses 12 einer erfindungsgemäßen Umschaltventilbaugruppe 10 erläutert.

In Figur 1 ist das Gehäuse 12 einer erfindungsgemäßen Umschaltventilbaugruppe perspektivisch von außen gezeigt. Der Betrachter von Figur 1 blickt auf eine Stirnseite als einen zweiten Außenseitenabschnitt 14. Der Außenseitenabschnitt 14 weist einen ebenen zweiten Stapel-Außenflächenabschnitt 16 auf, in welchem eine Ausgabe-Anschlussöffnung 18 ausgebildet ist. Die Ausgabe-Anschlussöffnung 18 bildet das Ende eines Ausgabe-Gaslieferleitungsabschnitts 20, welcher sich im dargestellten Beispiel längs einer virtuellen Ausgabe-Gaslieferbahn AG erstreckt, die aus Gründen vereinfachter Fertigung eine geradlinige Ausgabe-Gaslieferbahn AG ist. Die Ausgabe-Gaslieferbahn AG ist orthogonal zum Stapel-Außenflächenabschnitt 16 orientiert.

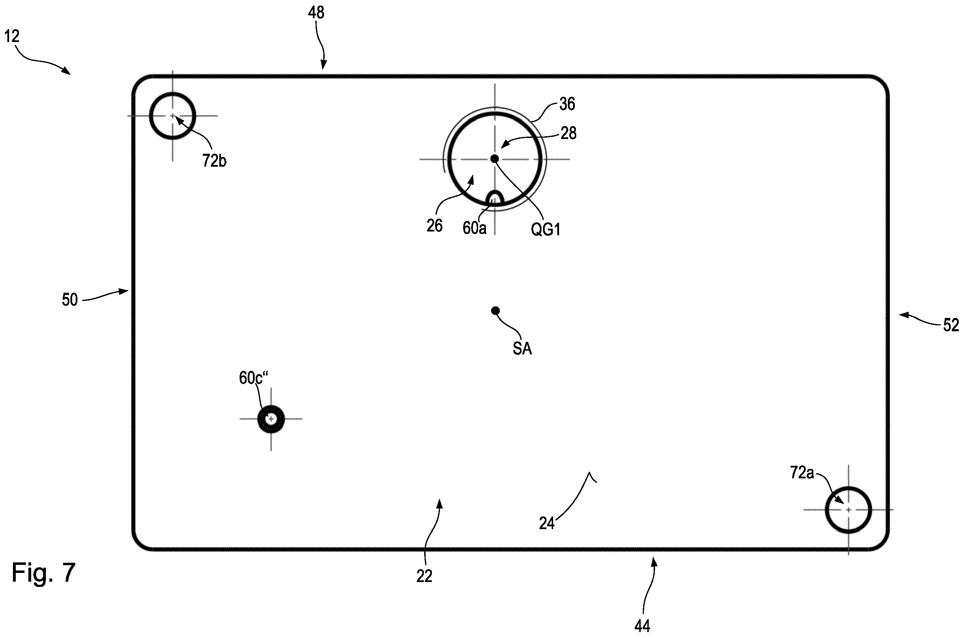

Figur 7 zeigt die den ersten Außenseitenabschnitt 22 bildende Stirnseite, welche einen ebenfalls ebenen ersten Stapel-Außenflächenabschnitt 24 aufweist. Die ebenen Stapel-Außenflächenabschnitte 24 und 16 sind zueinander parallel und sind flächig in Anlage aneinander bringbar, wenn zwei Gehäuse 12 mit je einem Stapel-Außenflächenabschnitt 24 und 16 verwendet werden.

Im ersten Außenseitenabschnitt 22 und auch im ersten Stapel-Außenflächenabschnitt 24 ist eine erste Quellen-Anschlussöffnung 26 ausgebildet, von welcher ausgehend ein erster Quellen-Gaslieferleitungsabschnitt 28 längs einer virtuellen ersten Quellen-Gaslieferbahn QG1 in das Gehäuse 12 hinein verläuft. Die erste Quellen-Gaslieferbahn QG1 ist geradlinig und zum ersten Stapel-Außenflächenabschnitt 24 orthogonal orientiert.

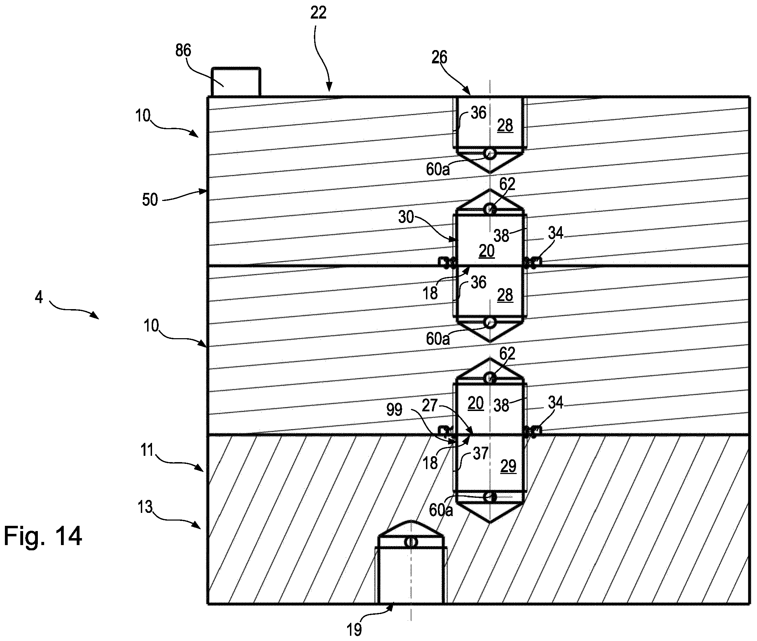

Die erste Quellen-Anschlussöffnung 26 und die Ausgabe-Anschlussöffnung 18 können dann, wenn zwei gleichartige Umschaltventilbaugruppen bzw. Gehäuse 12 längs einer Stapelachse SA in einem Stapeleingriff miteinander derart gestapelt sind, dass der erste Stapel-Außenflächenabschnitt 24 und der zweite Stapel-Außenflächenabschnitt 16 aneinander anliegen, miteinander fluchten. Die erste Quellen-Anschlussöffnung 26 ist daher eine Folge-Anschlussöffnung 26 im Sinne der vorstehenden Beschreibungseinleitung. Daher sind die erste Quellen-Anschlussöffnung 26 und die Ausgabe-Anschlussöffnung 18 längs zueinander koaxialer Gaslieferbahnen: erste Quellen-Gaslieferbahn Q1 und Ausgabe-Gaslieferbahn AG, angeordnet, welche eine gemeinsame Öffnungsachse bilden. Auch der erste Quellen-Gaslieferleitungsabschnitt 28 und der Ausgabe-Gaslieferleitungsabschnitt 20 fluchten im beschriebenen Stapeleingriff miteinander und bilden über die miteinander fluchtenden Anschlussöffnungen 26 und 18 hinweg einen gemeinsamen Gaslieferleitungsbereich 30 (siehe Figur 14).

Die Ausgabe-Anschlussöffnung 18 ist von einer Dichtungsanordnung 32 in Gestalt eines O-Rings umgeben, welcher in einer mit radialem Abstand von der Ausgabe-Anschlussöffnung 18 konzentrisch zur Ausgabe-Anschlussöffnung 18 ausgebildeten Nut 34 angeordnet ist. Im Stapeleingriff mit einem ersten Stapel-Außenflächenabschnitt 22 eines weiteren Gehäuses 12 liegt die Dichtungsanordnung 32 an dem ersten Stapel-Außenflächenabschnitt 24 dichtend an.

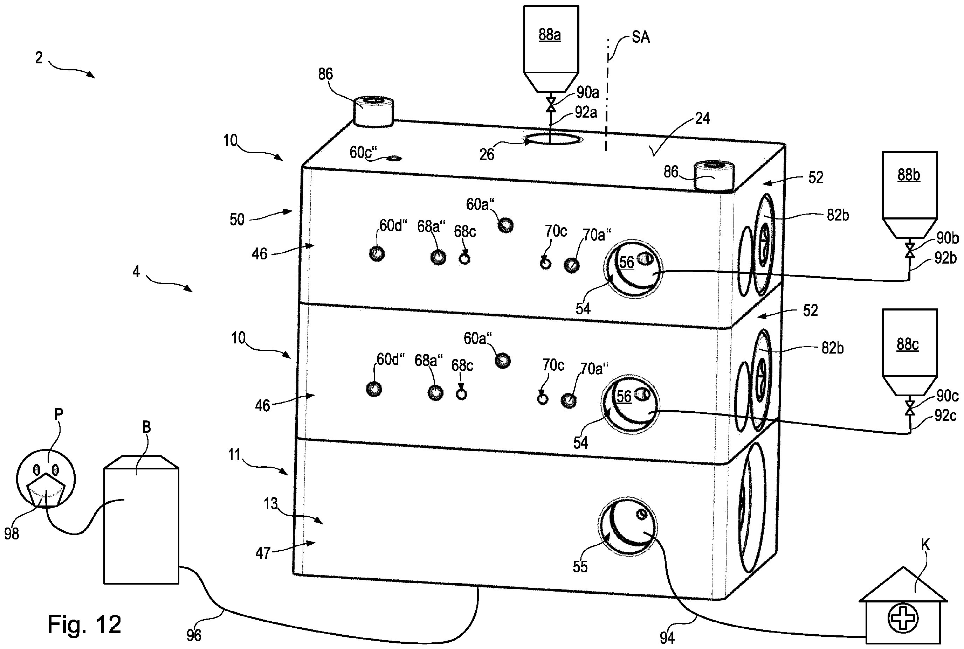

Zur Sicherung einer ersten Gasquelle 88a (s. Fig. 12), etwa eines von einer Gasflasche 88a zum Gehäuse 12 führenden Leitungsschlauchs 92a (s. Fig. 12), ist an der ersten Quellen-Anschlussöffnung 26 sowie im ersten Quellen-Gaslieferleitungsabschnitt 28 eine erste Quellen-Anschlussformation 36 in Gestalt eines Innengewindes ausgebildet. In dieses kann ein Verbindungsstutzen eines Leitungsschlauchs 92a o. ä. eingeschraubt werden.

Ebenso ist zur Sicherung einer zu einem Verbraucher führenden Gasleitung, wiederum etwa eines Leitungsschlauchs 96 (s. Fig. 12), an der Ausgabe-Anschlussöffnung 18 sowie im Ausgabe-Gaslieferleitungsabschnitt 20 eine Ausgabe-Anschlussformation 38, ebenfalls in Gestalt eines Innengewindes, ausgebildet. In dieses kann ein Verbindungsstutzen eines zu einem Verbraucher führenden Leitungsschlauchs 96 o. ä. eingeschraubt werden.

Anstelle der dargestellten Innengewinde können auch Bajonettverschluss-Formationen oder Pneumatik-Schnellkupplungen als Anschlussformationen ausgebildet sein.

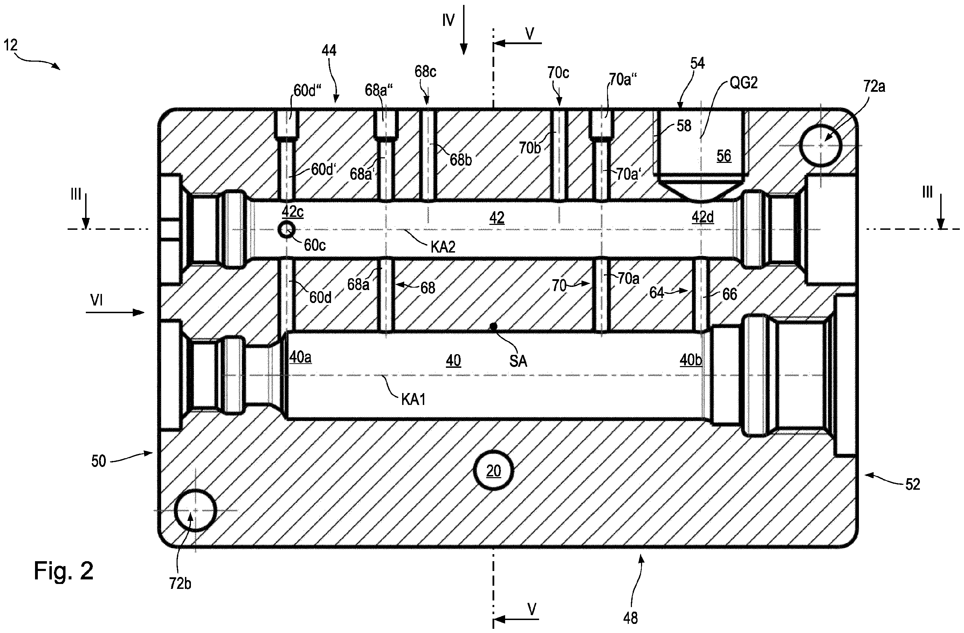

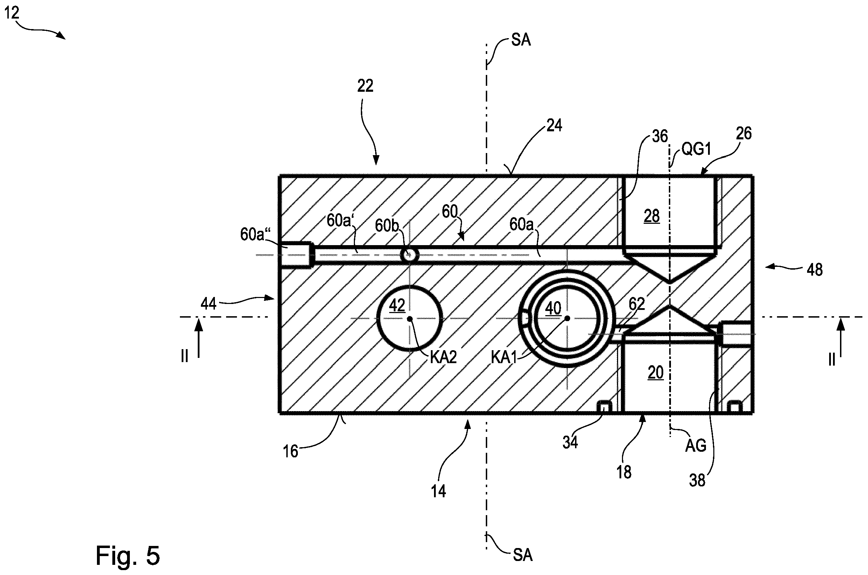

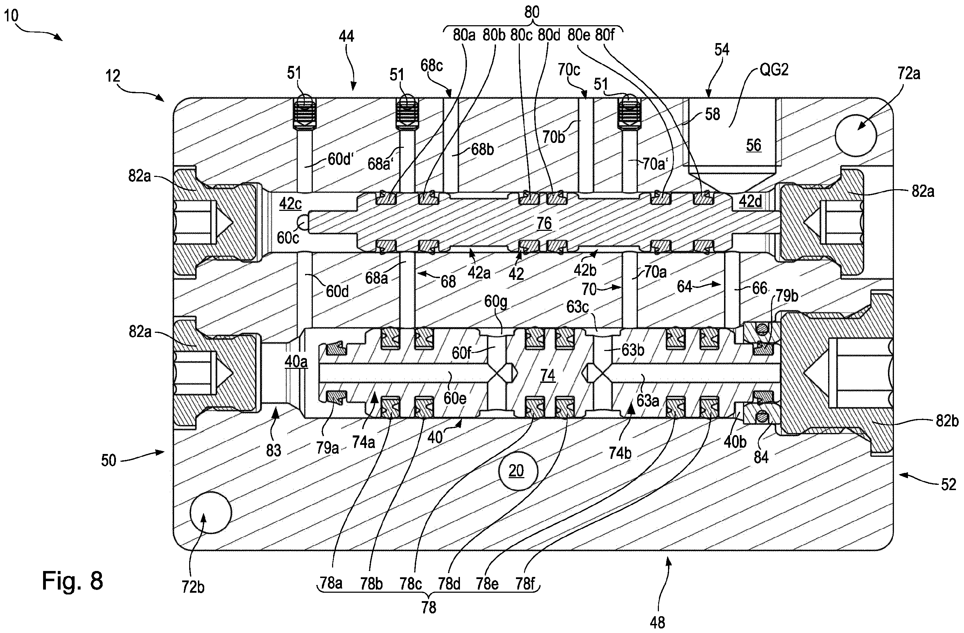

Wie in den Figuren 2 und 5 zu erkennen ist, weist das Gehäuse 12 eine erste, näher bei der Ausgabe-Anschlussöffnung 18 bzw. dem Ausgabe-Gaslieferleitungsabschnitt 20 gelegene Ventilkörperkavität 40 und eine zweite, weiter von der Ausgabe-Anschlussöffnung 18 bzw. dem Ausgabe-Gaslieferleitungsabschnitt 20 entfernt gelegene Ventilkörperkavität 42 auf. Die erste Ventilkörperkavität 40 erstreckt sich längs einer geradlinigen ersten Kavitätsachse KA1. Die zweite Ventilkörperkavität 42 erstreckt sich längs einer geradlinigen, zur ersten Kavitätsachse KA1 parallelen zweiten Kavitätsachse KA2. Die Parallelität der Kavitätsachsen KA1 und KA2 ist nicht zwingend notwendig, jedoch praktisch. In den Ventilkörperkavitäten 40 und 42 sind an der betriebsfertigen Umschaltventilbaugruppe 10 ein erster bzw. ein zweiter Ventilkörper 74, 76 verlagerbar aufgenommen (siehe Figuren 8 bis 11).

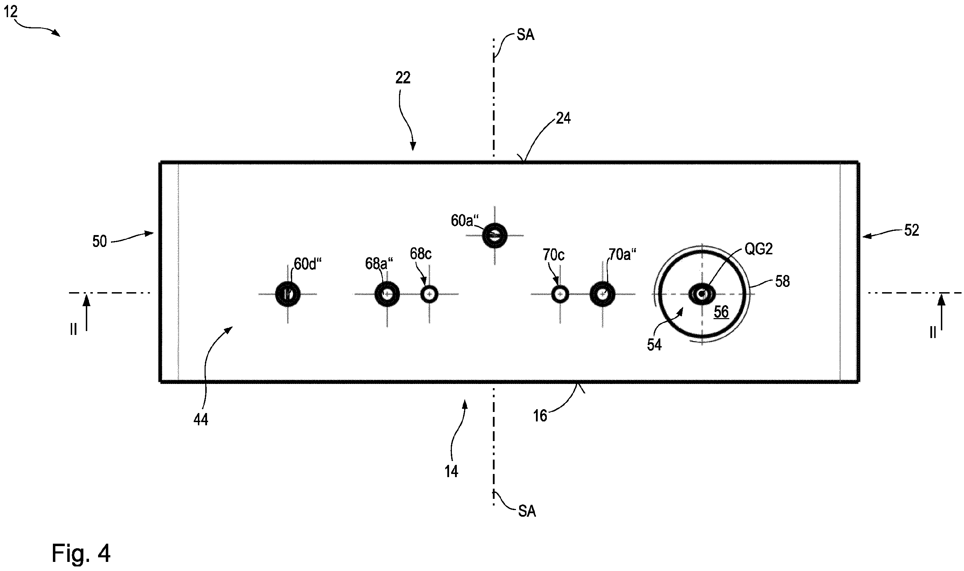

Der Betrachter von Figur 4 blickt auf den in Figur 1 vom Betrachter abgewandten Längsabschnitt 44 der zwischen den Stirnseiten umlaufenden Mantelfläche 46. Der Längsabschnitt 44 bildet einen dritten Außenseitenabschnitt, welcher zwischen dem ersten Außenseitenabschnitt 22 und dem zweiten Außenseitenabschnitt 16 verläuft, im dargestellten Beispiel des quaderförmigen Gehäuses 12 orthogonal zu diesen.

Parallel zu dem Längsabschnitt 44 verläuft der in Figur 1 erkennbare weitere Längsabschnitt 48. Die beiden Längsabschnitte 44 und 48 sind durch zwei zueinander ebenfalls parallele und zu den Stirnseiten orthogonale Querabschnitte 50 und 52 der Mantelfläche 46 miteinander verbunden.

In dem Längsabschnitt 44, also dem dritten Außenseitenabschnitt 44, ist eine zweite Quellen-Anschlussöffnung 54 ausgebildet, welche in ihrer Gestalt der ersten Quellen-Anschlussöffnung 26 gleicht. Dies hat den Vorteil, dass eine an die erste Quellen-Anschlussöffnung 26 anschließbare Gasquelle auch an der zweiten Quellen-Anschlussöffnung 54 anschließbar ist und umgekehrt. Von der zweiten Quellen-Anschlussöffnung 54 erstreckt sich somit ein zweiter Gaslieferleitungsabschnitt 56 entlang einer virtuellen zweiten Quellen-Gaslieferbahn QG2, welche wie die vorher genannten Gaslieferbahnen QG1 und AG als geradlinige Quellen-Gaslieferbahn QG2 ausgebildet ist. Die zweite Quellen-Gaslieferbahn QG2 ist im dargestellten Beispiel orthogonal zur ersten Quellen-Gaslieferbahn QG1 und zur Ausgabe-Gaslieferbahn AG orientiert. Zur Sicherung einer Verbindung zwischen einer Gasquelle, welche auch eine Gasleitung sein kann, und dem Gehäuse 12 ist im zweiten Gaslieferleitungsabschnitt 56 eine zweite Quellen-Anschlussformation 58 in Gestalt eines Innengewindes ausgebildet. Es gilt das zur ersten Quellen-Anschlussformation 36 Gesagte auch für die zweite Quellen-Anschlussformation 58.

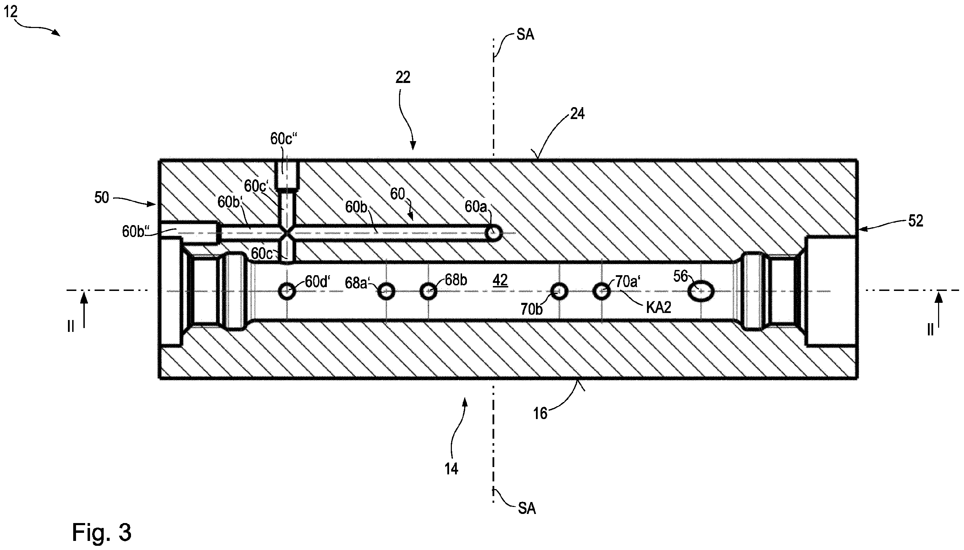

Nachfolgend wird die erste Gaslieferleitung 60 erläutert: die erste Gaslieferleitung 60, zu der bereits der erste Gaslieferleitungsabschnitt 28 gehört, umfasst im dargestellten Beispiel einen weiteren Leitungsabschnitt 60a, welcher sich vom ersten Gaslieferleitungsabschnitt 28 parallel zum ersten und zum zweiten Außenseitenabschnitt 24 bzw. 16 und außerdem parallel zu den Querabschnitten 50 und 52 der Mantelfläche 46 an den Ventilkörperkavitäten 40 und 42 vorbei erstreckt (siehe Figur 3). Hergestellt ist der weitere Leitungsabschnitt 60a beispielhaft als Bohrung, welche ausgehend vom Längsabschnitt 44 orthogonal zu diesem bis zum ersten Gaslieferleitungsabschnitt 28 geführt ist.

An den Leitungsabschnitt 60a schließt sich orthogonal zu diesem und parallel zur zweiten Ventilkörperkavität 42 bzw. parallel zur zweiten Kavitätsachse KA2 ein Leitungsabschnitt 60b an, welcher beispielhaft als Bohrung ausgehend vom Querabschnitt 50 ausgeführt ist.

Schließlich weist die erste Gaslieferleitung 60 einen sowohl zum Leitungsabschnitt 60a wie auch zum Leitungsabschnitt 60b orthogonalen dritten Leitungsabschnitt 60c auf, welcher schließlich in einen ersten, näher beim Querabschnitt 50 gelegenen Längsendbereich 42c der zweiten Ventilkörperkavität 42 mündet. Von dort aus führt ein weiterer Leitungsabschnitt 60d der ersten Gaslieferleitung 60 zu einem ersten, näher beim Querabschnitt 50 gelegenen Längsendbereich 40a der ersten Ventilkörperkavität 40. Von der ersten Ventilkörperkavität 40, und zwar etwa von deren Längsmitte, führt ein Leitungsabschnitt 62 (s. Fig. 5) der ersten Gaslieferleitung 60 zum Ausgabe-Gaslieferleitungsabschnitt 20. So kann also Gas von der ersten Quellen-Anschlussöffnung 22 längs der ersten Gaslieferleitung 60 bis zur Ausgabe-Anschlussöffnung 18 strömen.

Nachfolgend wird die zweite Gaslieferleitung 64 beschrieben (siehe Figur 2): der von der zweiten Quellen-Anschlussöffnung 54 sich ins Gehäuse 12 hinein erstreckende zweite Quellen-Gaslieferleitungsabschnitt 56 ist ein erster Abschnitt der zweiten Gaslieferleitung 56. Dieser zweite Quellen-Gaslieferleitungsabschnitt 56 mündet unmittelbar in einen näher bei dem Querabschnitt 52 gelegenen Längsendbereich 42d der zweiten Ventilkörperkavität 42. Von diesem Längsendbereich 42d der zweiten Ventilkörperkavität 42 führt ein weiterer Leitungsabschnitt 66 zu einem näher bei dem Querabschnitt 52 gelegenen Längsendbereich 40b der ersten Ventilkörperkavität 40. Von der ersten Ventilkörperkavität 40 führt der Leitungsabschnitt 62, der somit sowohl Teil der ersten Gaslieferleitung 60 als auch Teil der zweiten Gaslieferleitung 64 ist, zum Ausgabe-Gaslieferleitungsabschnitt 20, welcher ebenfalls Teil sowohl der ersten Gaslieferleitung 60 als auch der zweiten Gaslieferleitung 64 ist.

Der Leitungsabschnitt 62 liegt vor den Schnittebenen der Figuren 2 und 8 bis 11.

Die Leitungsabschnitte 60d und 66 verlaufen somit parallel zueinander zwischen entgegengesetzten Längsendbereichen der ersten Ventilkörperkavität 40 und der zweiten Ventilkörperkavität 42.

Im Gehäuse 12 ist außerdem eine erste Entlüftungsleitung 68 und eine zweite Entlüftungsleitung 70 ausgebildet. Die erste Entlüftungsleitung 68 befindet sich näher beim Querabschnitt 50 und verläuft bevorzugt parallel zu diesem sowie parallel zu den Stirnseiten. Die zweite Entlüftungsleitung 70 befindet sich näher beim Querabschnitt 52 und verläuft bevorzugt parallel zu diesem sowie parallel zu den Stirnseiten.

Die erste Entlüftungsleitung 68 umfasst einen ersten Leitungsabschnitt 68a, welcher bevorzugt parallel zum Leitungsabschnitt 60d, jedoch näher bei der Längsmitte der ersten Ventilkörperkavität 40 zwischen der ersten Ventilkörperkavität 40 und der zweiten Ventilkörperkavität 42 verläuft und die Kavitäten miteinander verbindet.

Die erste Entlüftungsleitung 68 umfasst außerdem einen zweiten Leitungsabschnitt 68b, welcher von der zweiten Ventilkörperkavität 42 zu einer Senken-Anschlussöffnung 68c verläuft und damit zur Außenumgebung des Gehäuses 12 hin öffnet. Im dargestellten Beispiel befindet sich die Senken-Anschlussöffnung 68c im Längsabschnitt 44 der Mantelfläche 46. Die Mündung des zweiten Leitungsabschnitts 68b in die zweite Ventilkörperkavität 42 ist relativ zur Mündung des ersten Leitungsabschnitts 68a in die zweite Ventilkörperkavität 42 längs der zweiten Kavitätsachse KA2 versetzt angeordnet, und zwar im dargestellten Beispiel in Richtung zur Längsmitte der zweiten Ventilkörperkavität 42 hin.

Die zweite Entlüftungsleitung 70 umfasst einen ersten Leitungsabschnitt 70a, welcher bevorzugt parallel zum Leitungsabschnitt 66, jedoch näher bei der Längsmitte der ersten Ventilkörperkavität 40 zwischen der ersten Ventilkörperkavität 40 und der zweiten Ventilkörperkavität 42 verläuft und die Kavitäten miteinander verbindet.

Die zweite Entlüftungsleitung 70 umfasst außerdem einen zweiten Leitungsabschnitt 70b, welcher von der zweiten Ventilkörperkavität 42 zu einer Senken-Anschlussöffnung 70b verläuft und damit zur Außenumgebung des Gehäuses 12 hin öffnet. Im dargestellten Beispiel befindet sich die Senken-Anschlussöffnung 70c im Längsabschnitt 44 der Mantelfläche 46. Die Mündung des zweiten Leitungsabschnitts 70b in die zweite Ventilkörperkavität 42 ist relativ zur Mündung des ersten Leitungsabschnitts 70a in die zweite Ventilkörperkavität 42 längs der zweiten Kavitätsachse KA2 versetzt angeordnet, und zwar im dargestellten Beispiel in Richtung zur Längsmitte der zweiten Ventilkörperkavität 42 hin.

Die beiden zweiten Abschnitte 68b und 70b der ersten bzw. der zweiten Entlüftungsleitung 68 bzw. 70 weisen folglich einen geringeren Abstand längs der zweiten Kavitätsachse KA2 voneinander auf als die ersten beiden Abschnitte 68a und 70a.

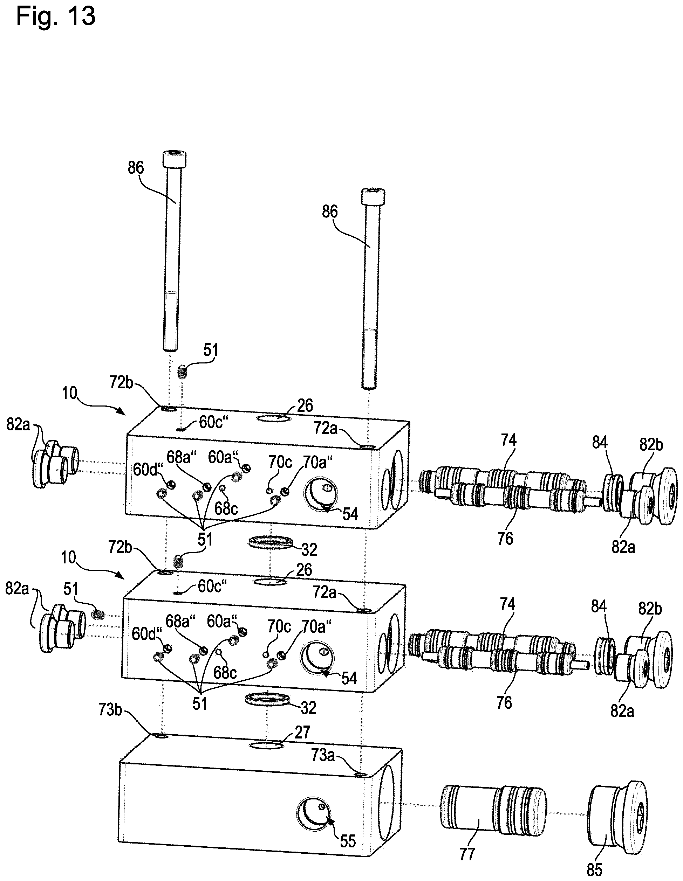

An zwei einander diagonal gegenüberliegenden Ecken der insgesamt vier Ecken des Gehäuses 12 ist je eine Verbindungsformation 72a bzw. 72b in beispielhafter Gestalt einer Durchgangsbohrung bzw. eines Durchgangskanals ausgebildet. Durch die Verbindungsformationen 72a und 72b kann je beispielsweise eine Schraube 86 (s. Fig. 12 und 13) oder eine Gewindestange oder eine andere Spannvorrichtung hindurchgeführt werden, um zwei oder mehr Gehäuse 12 miteinander längs einer zu den Stirnseiten orthogonalen Stapelachse SA zu verbinden und aneinander zu sichern. Die Verbindungsformation 72a befindet sich dabei in der vom Querabschnitt 52 und vom Längsabschnitt 44 gebildeten Gehäuseecke. Die Verbindungsformation 72b befindet sich in der gegenüberliegenden, vom Querabschnitt 50 und vom Längsabschnitt 48 gebildeten Gehäuseecke. Die Verbindungsformationen können in mehr als zwei Ecken, in einem anderen Eckenpaar oder auch an anderen Orten als Gehäuseecken ausgebildet sein, um nur einige mögliche Variationen zu nennen.

Ganz allgemein sind jene in der Verlängerung von Leitungsabschnitten gelegenen Abschnitte, welche selbst keine Leitungsfunktion erfüllen, sondern nur zur Herstellung des zugeordneten koaxialen Leitungsabschnitts gebildet wurden, mit dem gleichen Bezugszeichen wie der zugeordnete koaxiale Leitungsabschnitt bezeichnet, jedoch unter Hinzufügung eines Apostrophen. Diese apostrophierten Fertigungsabschnitte sind an dem Außenseitenabschnitt, in welchen sie münden, mit einem endabschnitt mit vergrößertem Durchmesser ausgebildet, um darin einen Verschluss 51 der apostrophierten Fertigungsabschnitte anordnen zu können, welcher die Fertigungsabschnitte gegen die Außenumgebung abgedichtet. Die Endabschnitte mit vergrößertem Durchmesser sind mit dem gleichen Bezugszeichen wie der zugeordnete koaxiale Leitungsabschnitt bezeichnet, jedoch unter Hinzufügung eines Doppel-Apostrophen.

In Figur 8 ist die vollständige Umschaltventilbaugruppe 10 gezeigt, mit einem in der ersten Ventilkörperkavität 40 aufgenommenen ersten Ventilkörper 74 mit einem in der zweiten Ventilkörperkavität 42 aufgenommenen zweiten Ventilkörper 76. Der erste und der zweite Ventilkörper 74 bzw. 76 sind translatorisch verlagerbare, doppeltwirkende Ventilkolben, welche an ihren jeweiligen Längsenden mit Gas aus der ersten Gaslieferleitung 60 und aus der zweiten Gaslieferleitung 64 beaufschlagt sind. In Figur 8 ist die Umschaltventilbaugruppe 10 in ihrer ersten Betriebsstellung dargestellt, in welcher die erste Gaslieferleitung 60 zur Durchströmung mit Gas von der ersten Quellen-Anschlussöffnung 26 zur Ausgabe-Anschlussöffnung 18 freigegeben ist und in welcher die zweite Gaslieferleitung 64 für eine Durchströmung mit Gas von der zweiten Quellen-Anschlussöffnung 54 zur Ausgabe-Anschlussöffnung 18 gesperrt ist. Ebenso sind die erste Entlüftungsleitung 68 gesperrt und die zweite Entlüftungsleitung 70 für eine Durchströmung geöffnet.

Der erste Ventilkörper 74 und der zweite Ventilkörper 76 befinden sich in ihrer ersten Betriebsstellung.

Der erste Ventilkörper 74 weist eine erste Dichtungsanordnung 78 auf, welche im dargestellten Beispiel von links nach rechts sechs Dichtungsringe 78a bis 78f umfasst. Die Dichtungsringe 78a bis 78f sind zur gemeinsamen Bewegung mit dem ersten Ventilkörper 74 am ersten Ventilkörper 74 angeordnet und dichten gegen die Innenwandung der ersten Ventilkörperkavität 40.

Ebenso weist der zweite Ventilkörper 76 eine zweite Dichtungsanordnung 80 auf, welche im dargestellten Beispiel von links nach rechts sechs Dichtungsringe 80a bis 80f umfasst. Die Dichtungsringe 80a bis 80f sind zur gemeinsamen Bewegung mit dem zweiten Ventilkörper 76 am zweiten Ventilkörper 76 angeordnet und dichten gegen die Innenwandung der zweiten Ventilkörperkavität 42.

Die erste Dichtungsanordnung 78, insbesondere die Dichtungen 78c und 78d, definiert in der ersten Ventilkörperkavität 40 den ersten Kavitätsbereich 40a und den vom ersten strömungsmechanisch getrennten zweiten Kavitätsbereich 40b. Der erste Kavitätsbereich 40a ist als ein erster Liefer-Kavitätsbereich 40a Teil der ersten Gaslieferleitung 60, aber nicht der zweiten Gaslieferleitung 64. Der zweite Kavitätsbereich 40b ist als ein zweiter Liefer-Kavitätsbereich 40b Teil der zweiten Gaslieferleitung 64, aber nicht der ersten Gaslieferleitung 60.

Im ersten Ventilkörper 74 sind an dessen in Figur 8 linkem Ende, im ersten Liefer-Ventilkörperbereich 74a des ersten Ventilkörpers 74, ein längs der Längsachse des ersten Ventilkörpers 74 verlaufender Leitungsabschnitt 60e und ein orthogonal hierzu verlaufender Leitungsabschnitt 60f ausgebildet. Der Leitungsabschnitt 60f mündet in eine in Umfangsrichtung um die Längsachse des ersten Ventilkörpers 74 umlaufende Ringleitung 60g. Die im oder am ersten Ventilkörper 74 ausgebildeten Leitungsabschnitte 60e, 60f und 60g sind sowohl Teil der ersten Gaslieferleitung 60 als auch Teil der ersten Entlüftungsleitung 68.

Ebenso sind im ersten Ventilkörper 74, welcher bezüglich einer Längsmittelebene spiegelbildlich ausgebildet ist, an dessen in Figur 8 rechtem Ende, in einem zweiten Liefer-Ventilkörperbereich 74b des ersten Ventilkörpers 74, ein längs der Längsachse des ersten Ventilkörpers 74 verlaufender Leitungsabschnitt 63a und ein orthogonal hierzu verlaufender Leitungsabschnitt 63b ausgebildet. Der Leitungsabschnitt 63b mündet in eine in Umfangsrichtung um die Längsachse des ersten Ventilkörpers 74 umlaufende Ringleitung 63c. Die im oder am ersten Ventilkörper 74 ausgebildeten Leitungsabschnitte 63a, 63b und 63c sind sowohl Teil der zweiten Gaslieferleitung 64 als auch Teil der zweiten Entlüftungsleitung 70.

An der fertigen Umschaltventilbaugruppe 10 sind die Ventilkörperkavitäten 40 und 42 an ihren jeweiligen Längsenden mit Verschlussmuttern 82a und 82b verschlossen. Die drei Verschlussmuttern 82a sind identisch. Die Verschlussmutter 82b ist größer als die Verschlussmutter 82a, da durch die von ihr verschlossene Öffnung der erste Ventilkörper 74 in die erste Ventilkörperkavität 40 eingeführt wird. Aus demselben Grunde ist eine an dem in Figur 8 linken Ende der ersten Ventilkörperkavität 40 einstückig mit dem Gehäuse 12 ausgebildete Engstelle 83, welche einen kleineren Durchmesser als der übrige mittlere Bereich der ersten Ventilkörperkavität 40 aufweist, an dem in Figur 8 rechten Ende der ersten Ventilkörperkavität 40 durch eine gesondert ausgebildete und montierte Buchse 84 nachgebildet.

Die Dichtungsanordnung 78, insbesondere die Dichtungen 78a und 78b einerseits sowie die Dichtungen 78e und 78f andererseits, grenzen in der in Figur 8 gezeigten ersten Betriebsstellung des ersten Ventilkörpers 74 den Leitungsabschnitt 68a der ersten Entlüftungsleitung 68 von der übrigen ersten Ventilkörperkavität 40 ab und unterbinden somit eine Gasströmung von der ersten Ventilkörperkavität 40 zu in Entlüftungsrichtung stromabwärts gelegenen Leitungsabschnitten der ersten Entlüftungsleitung 68.

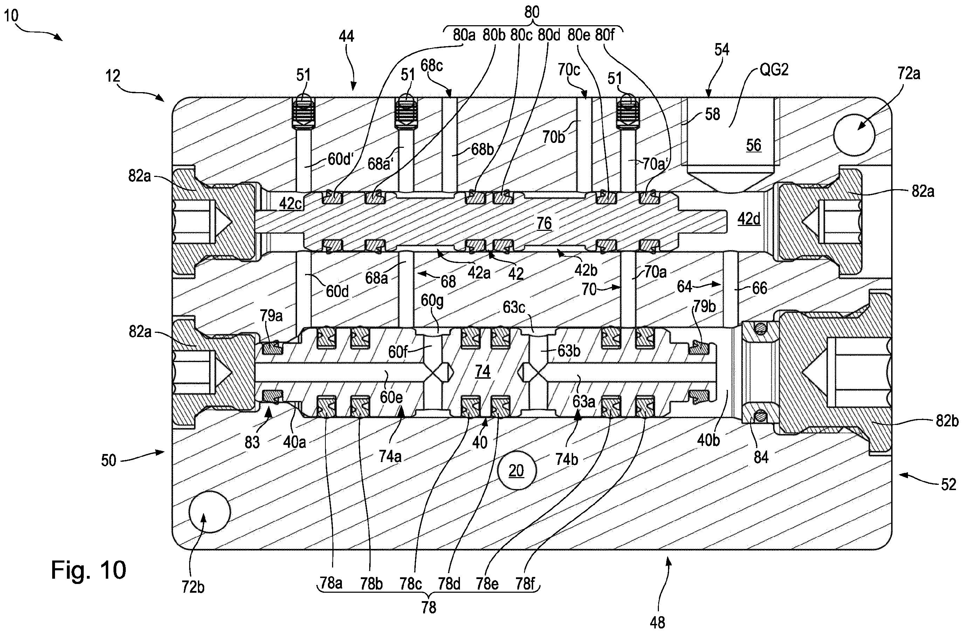

Die Dichtungsanordnung 78, insbesondere die Dichtungen 78e und 78f machen in der zweiten Betriebsstellung des ersten Ventilkörpers 74 (siehe Figur 10) das gleiche mit dem Leitungsabschnitt 70a bzw. mit den in Entlüftungsrichtung stromabwärts der ersten Ventilkörperkavität 40 gelegenen Leitungsabschnitten der zweiten Entlüftungsleitung 70. In der in Figur 8 gezeigten ersten Betriebsstellung des ersten Ventilkörpers 74 haben die Dichtungen 78e und 78f keine bestimmte Funktion.

Die zweite Dichtungsanordnung 80, insbesondere die Dichtungen 80c und 80d, grenzt am zweiten Ventilkörper 76 in dessen erstem Entlüftungs-Ventilkörperbereich 76a bzw. in der zweiten Ventilkörperkavität 42 einen dritten Kavitätsbereich 42a und einen in einem zweiten Entlüftungs-Ventilkörperbereich 76b des zweiten Ventilkörpers 76 gelegenen vierten Kavitätsbereich 42b voneinander ab. Der dritte Kavitätsbereich 42a ist als erster Entlüftungs-Kavitätsbereich 42a Teil der ersten Entlüftungsleitung 68, aber ist kein Teil der zweiten Entlüftungsleitung 70 oder einer der Gaslieferleitungen 60 und 64. Der vierte Kavitätsbereich 42b ist als zweiter Entlüftungs-Kavitätsbereich 42b Teil der zweiten Entlüftungsleitung 70, aber ist kein Teil der ersten Entlüftungsleitung 68 oder einer der Gaslieferleitungen 60 und 64. Die Kavitätsbereiche 42a und 42b sind axial mit Abstand voneinander ausgebildet und laufen jeweils ringförmig um den zweiten Ventilkörper 76 um.

Die zweite Dichtungsanordnung 80, insbesondere die Dichtung 80a einerseits und die Dichtung 80f andererseits, grenzen die längsendseitigen Kavitätsbereiche 42c und 42d vom dritten und vom vierten Kavitätsbereich 42a bzw. 42b ab. Die längsendseitigen Kavitätsbereiche 42c und 42d sind Teil der ersten Gaslieferleitung 60 bzw. der zweiten Gaslieferleitung 64 und sind unabhängig von der Stellung des zweiten Ventilkörpers 76 stets von der Quellen-Anschlussöffnung, in welcher sie enden, zur Ausgabe-Anschlussöffnung 18 hin durchströmbar. Der zweite Ventilkörper 76 sperrt also im dargestellten bevorzugten Ausführungsbeispiel in keiner seiner Betriebsstellungen eine Gaslieferleitung 60 oder 64, sondern gibt lediglich eine Entlüftungsleitung zur Durchströmung frei und sperrt die jeweils andere Entlüftungsleitung.

In der in Figur 8 gezeigten ersten Betriebsstellung des zweiten Ventilkörpers 76 grenzt die erste Dichtungsanordnung 80, insbesondere die Dichtungen 80a und 80b, den Leitungsabschnitt 68a der ersten Entlüftungsleitung 68 von der übrigen Ventilkörperkavität 42 ab und trennt den Leitungsabschnitt 68a somit vom Leitungsabschnitt 68b, wodurch die erste Entlüftungsleitung 68 durch den zweiten Ventilkörper 76 unterbrochen ist. Dagegen kann Gas aus dem zweiten Kavitätsbereich 40b über die Leitungsabschnitte 63a, 63b und die Ringleitung 63c in den Leitungsabschnitt 70a und von dort über den vierten Kavitätsbereich 42b in den Leitungsabschnitt 70b und schließlich über die Senken-Auslassöffnung 70c aus dem Gehäuse 12 in die Umgebungsatmosphäre entweichen.

In der dargestellten ersten Betriebsstellung des zweiten Ventilkörpers 76 kann im zweiten Kavitätsbereich 40b somit kein Gaspolster eingeschlossen werden, welches geeignet wäre, den ersten Ventilkörper 74 aus seiner in Figur 8 gezeigten ersten Betriebsstellung hinaus in Richtung zu seiner zweiten Betriebsstellung nach links zu drängen.

In der ersten Betriebsstellung des ersten Ventilkörpers 76 kann Gas von der ersten Quellen-Anschlussöffnung 26 über die Leitungsabschnitte 60a, 60b, 60c, den längsendseitigen Kavitätsbereich 42c, den Leitungsabschnitt 60d, den ersten Kavitätsbereich 40a, sowie über die im Inneren des ersten Ventilkörpers 74 ausgebildeten Leitungsabschnitte 60e und 60f sowie über die Ringleitung 60g in den Leitungsabschnitt 20 und somit in den Gaslieferleitungsabschnitt 20 und zur Ausgabe-Auslassöffnung 18 gelangen.

Die endseitigen Kolbenflächen des ersten Ventilkörpers 74 sind jeweils zweiteilig ausgebildet, wobei ein radial innerer, scheibenförmiger Kolbenflächenabschnitt mit axialem Abstand von einem radial äußeren ringförmigen Kolbenflächenabschnitt angeordnet ist. Axial zwischen den beiden Kolbenflächenabschnitten ein und derselben Kolbenfläche ist jeweils eine Dichtung 79a bzw. 79b angeordnet. Die Dichtung 79a liegt in der zweiten Betriebsstellung des ersten Ventilkörpers 74 an der Engstelle 83 an und unterbricht gemeinsam mit der Dichtungsanordnung 78, insbesondere der Dichtung 78a, in der zweiten Betriebsstellung die erste Gaslieferleitung 60. In der in Figur 8 dargestellten ersten Betriebsstellung des ersten Ventilkörpers 74 liegt die Dichtung 79b an einer Innenumfangsfläche der Buchse 84 an und sperrt somit gemeinsam mit der Dichtungsanordnung 78, insbesondere der Dichtung 78f, die zweite Gaslieferleitung 64. Gas von der zweiten Quellen-Anschlussöffnung 54 kann in der in Figur 8 gezeigten ersten Betriebsstellung des ersten Ventilkörpers 74 nur bis in den zweiten Kavitätsbereich 40b strömen, aber nicht weiter.

Durch die Aufteilung der beiden Kolbenflächen und durch die Abtrennung des radial inneren scheibenförmigen Kolbenflächenabschnitts durch die Dichtung 79a bzw. 79b auf der Seite der jeweils deaktivierten, gesperrten Gaslieferleitung wird eine Hysterese des ersten Ventilkörpers 74 bewirkt, die ein "Flattern" des ersten Ventilkörpers 74 vermeidet, wenn in den Gaslieferleitungen 60 und 64 etwa betragsmäßig gleiche Drücke herrschen.

Die beiden Ringleitungen 60g und 63c sind jeweils so gestaltet, dass sie in der einen der beiden Betriebsstellungen des ersten Ventilkörpers 74 eine Strömungsverbindung mit dem vor der Zeichenebene der Figuren 2 und 8 bis 11 gelegenen Leitungsabschnitt 62 herstellen und in der jeweils anderen der beiden Betriebsstellungen eine Strömungsverbindung mit jeweils einem anderen der Leitungsabschnitte 68a und 70a herstellen.

Ähnlich wie am ersten Ventilkörper 74 sind auch die Kolbenflächen an den Längsendbereichen des zweiten Ventilkörper 76 zweiteilig ausgebildet. Jede der Kolbenflächen weist einen radial inneren scheibenförmigen Kolbenflächenabschnitt und einen axial zu diesem versetzten radial äußeren ringförmigen Kolbenflächenabschnitt auf. Auch hierdurch wird eine Hysterese des zweiten Ventilkörpers 76 für den Fall erreicht, dass in den beiden endseitigen Kavitätsbereichen 42c und 42d betragsmäßig ähnliche oder gleiche Gasdrücke herrschen. Derjenige radial innere scheibenförmige Kolbenflächenabschnitt, welcher in einer Betriebsstellung des zweiten Ventilkörpers 76 an einer Verschlussmutter 82a anliegt, wird von Gas im jeweiligen Kavitätsbereich 42c oder 42d nicht erreicht, sodass der dort herrschende Gasdruck nur auf den radial äußeren ringförmigen Kolbenflächenabschnitt wirkt. Auf der entgegengesetzten Seite des zweiten Ventilkörpers 76 wirkt der Gasdruck dagegen auf die vollständige Kolbenfläche. Außerdem ist durch die den axialen Versatz der Kolbenflächenabschnitte sicherstellenden axialen endseitigen Zapfen sichergestellt, dass die endseitigen Kavitätsbereiche 42c und 42d unabhängig von der Stellung des zweiten Ventilkörpers 76 als Teil der ersten bzw. der zweiten Gaslieferleitung 60 bzw. 64 stets durchströmbar sind.

In der Darstellung von Figur 8 sei angenommen, dass an die erste Quellen-Anschlussöffnung 26 und an die zweite Quellen-Anschlussöffnung 54 jeweils eine volle Flasche mit Atemgas bzw. Sauerstoff zur Versorgung eines Patienten angeschlossen ist. Die an die zweite Quellen-Anschlussöffnung 54 angeschlossene Gasflasche wurde zuletzt gewechselt. Während dieses Austauschvorgangs wurde Gas von der Flasche an der ersten Quellen-Anschlussöffnung 26 über die erste Gaslieferleitung 60 zur Ausgabe-Anschlussöffnung 18 geliefert. Dieser Lieferzustand hält nach wie vor an.

In der ersten Gaslieferleitung 60 und in der zweiten Gaslieferleitung 64 herrschen somit etwa gleiche Gasdrücke. Da jedoch der Gasdruck in der ersten Gaslieferleitung 60 jeweils auf die vollständige linke Kolbenfläche des ersten und des zweiten Ventilkörpers 74 bzw. 76 wirken, während der Gasdruck in der zweiten Gaslieferleitung 64 nur auf die radial äußeren ringförmigen rechten Kolbenflächenabschnitte des ersten und des zweiten Ventilkörpers 74 bzw. 76 wirkt, resultiert an beiden Ventilkörpern 74 und 76 jeweils eine Kraft, welche jeden der Ventilkörper 74 und 76 nach rechts in die in Figur 8 gezeigte und bereits eingenommene erste Betriebsstellung belastet bzw. spannt.

Bevorzugt sind Gasflaschen, also einzelne Gasflaschen, über einen Druckminderer 90a, 90b, 90c (s. Fig. 12) an die Quellen-Anschlussöffnungen 26 und 54 angeschlossen, welcher den in den Gasflaschen herrschenden höheren Druck auf beispielsweise einen Druck von 6 bar (600 kPa) reduziert. Dieser an den Quellen-Anschlussöffnungen 26 und 54 herrschende reduzierte Druck bleibt bis kurz vor der vollständigen Entleerung in etwa konstant, bevor er dann mit Annäherung an die vollständige Entleerung der Flasche rasch abzufallen beginnt.

Die in Figur 8 Gas liefernde Quelle an der ersten Quellen-Anschlussöffnung 26 geht nun zur Neige, was zur Folge hat, dass der in der zweiten Gaslieferleitung 64 herrschende Gasdruck relativ zu dem in der ersten Gaslieferleitung 60 herrschenden Gasdruck ansteigt. Die Kolbenflächen der Ventilkörper 74 und 76 sind so bemessen, dass ein geringerer Druckunterschied zwischen der ersten und der zweiten Gaslieferleitung 60 bzw. 64 ausreicht, den zweiten Ventilkörper 76 zwischen seinen Betriebsstellungen zu verlagern, als für eine Verlagerung des ersten Ventilkörpers 74 zwischen dessen Betriebsstellungen benötigt wird.

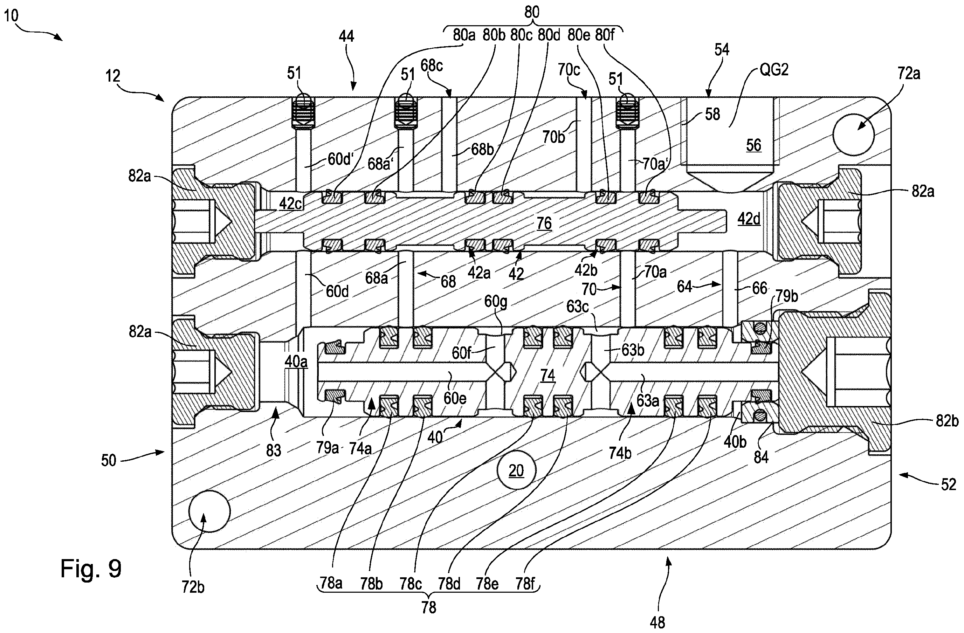

Figur 9 zeigt eine Übergangsphase bei abfallendem Gasdruck in der ersten Gaslieferleitung 60, in welcher der relative Überdruck in der zweiten Gaslieferleitung 64 bezüglich der ersten Gaslieferleitung 60 ausreicht, den zweiten Ventilkörper 76 von dessen erster Betriebsstellung in die zweite zu verlagern, jedoch nicht ausreicht, um auch den ersten Ventilkörper 74 von dessen erster Betriebsstellung in die zweite zu verlagern.

In der in Figur 9 gezeigten zweiten Betriebsstellung des zweiten Ventilkörpers 76 sperrt dieser die zweite Entlüftungsleitung 70 und öffnet die erste Entlüftungsleitung 78, sodass dann, wenn auch der erste Ventilkörper 74 in die zweite Betriebsstellung verlagert wird, Gas aus dem ersten Kavitätsbereich 40a über die erste Entlüftungsleitung 68 zur ersten Senken-Auslassöffnung 68c und somit zur Außenumgebung hin entweichen kann. Dies kann jedoch erst erfolgen, wenn der erste Ventilkörper 76 seine zweite Betriebsstellung erreicht und dadurch den ersten Kavitätsbereich 40a über die Leitungsabschnitte 60e, 60f und die Ringleitung 60g mit dem Leitungsabschnitt 68a verbindet.

Sobald die Dichtung 79a gegen die Engstelle 83 abgedichtet, wird nur noch der radial äußere ringförmige Kolbenflächenabschnitt der linken Kolbenfläche des ersten Ventilkörpers 74 mit dem ohnehin abfallenden Druck der ersten Gaslieferleitung 60 beaufschlagt. Das dann bei fortgesetzter Bewegung des ersten Ventilkörpers 74 nach links im Bereich der darin ausgebildeten Leitungsabschnitte 60e, 60f und 60g eingeschlossene Gas wird mit dem ersten Ventilkörper 74 mitbewegt. Lediglich zwischen dem gegen die Engstelle 83 abgedichteten radial inneren Kolbenflächenabschnitt und der gegenüberliegenden Verschlussmutter 82a eingeschlossenes Gas wird durch die Bewegung des ersten Ventilkörpers 74 nach links verdichtet und sorgt für eine Erhöhung des Gasdrucks in den Leitungsabschnitten 60e, 60f und 60g im Inneren des ersten Ventilkörpers 74.

Sobald der erste Ventilkörper 74 seine in Figur 10 gezeigte zweite Betriebsstellung erreicht hat, kann überschüssiges Gas aus den Leitungsabschnitten 60e, 60f und 60g im Inneren des ersten Ventilkörpers 74 über die vom zweiten Ventilkörper 76 bereits geöffnete erste Entlüftungsleitung 68 entweichen.

Da in Figur 10 sich beide Ventilkörper 74 und 76 in der zweiten Betriebsstellung befinden, zeigt Figur 10 die Umschaltventilbaugruppe 10 in ihrem zweiten Betriebszustand. Die erste Gaslieferleitung 60 ist nun unterbrochen und die an der ersten Quellen-Anschlussöffnung 26 angeschlossene entleerte Quelle kann gegen eine frische, gefüllte Quelle ausgetauscht werden. Aufgrund der beschriebenen Aufteilung der Kolbenflächen und der im zweiten Betriebszustand auf der linken Seite von Figur 10 von Gas in der ersten Gaslieferleitung 60 nur erreichbaren radial äußeren Kolbenflächenabschnitte ändert die durch den Quellentausch bewirkte Druckerhöhung in der ersten Gaslieferleitung 60 nichts am Betriebszustand der Umschaltventilbaugruppe 10. Diese liefert unterbrechungsfrei Gas von einer der Quellen bis zu deren Entleerung und gestattet dann deren Austausch durch eine gefüllte Quelle.

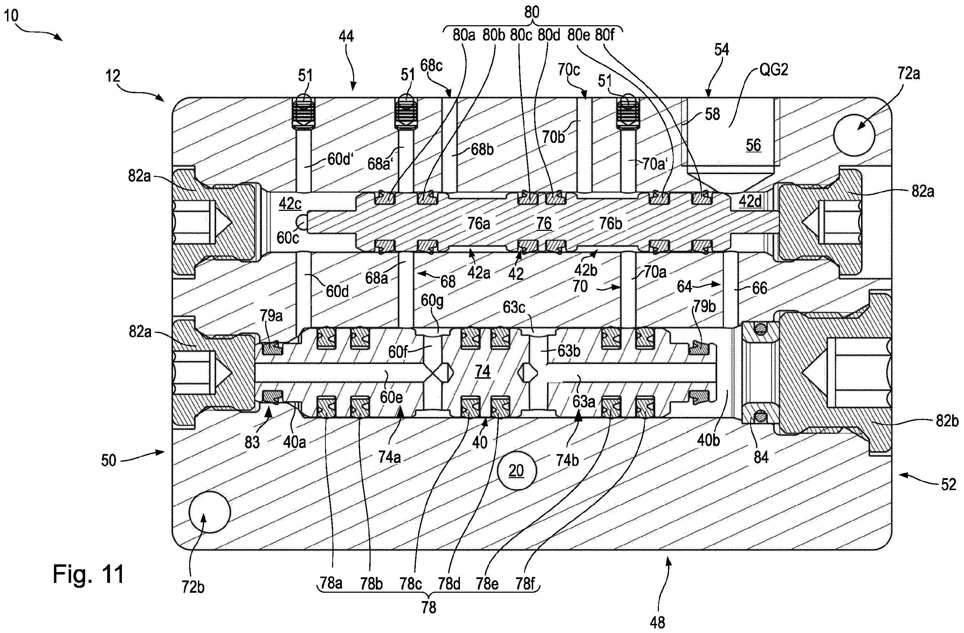

In Figur 11 ist eine der Übergangsphase von Figur 9 entsprechende weitere Übergangsphase der Umschaltventilbaugruppe 10 gezeigt, diesmal jedoch bei einem Übergang vom zweiten Betriebszustand in dem in Figur 8 gezeigten ersten Betriebszustand.

Bei fortschreitender Entleerung der an die zweite Quellen-Anschlussöffnung 54 angeschlossenen Quelle sorgt der relative Anstieg des Gasdrucks in der von einer frischen Quelle versorgten ersten Gaslieferleitung 60 zunächst für eine Verstellung des zweiten Ventilkörpers 76 in die erste Betriebsstellung, in welcher, wie in Figur 8, die erste Entlüftungsleitung 68 unterbrochen ist und die zweite Entlüftungsleitung für eine Entlüftung des zweiten Kavitätsbereichs 40b freigegeben ist.

In Figur 12 ist grobschematisch eine beispielhafte Anlage 2 zur Versorgung eines Patienten P mit Sauerstoff dargestellt. Die Anlage 2 umfasst einen Stapel 4 oder ein Paket 4 mit zwei Umschaltventilbaugruppen 10 und einer ebenfalls stapelbaren Priorisierungsventilbaugruppe 11, welche längs einer Stapelachse SA gestapelt sind.

Der Stapel 4 ist durch zwei Verbindungsschrauben 86 gegen ein Auseinanderfallen gesichert. Die Verbindungsschrauben 86 sind in die Durchgangsöffnungen 72a und 72b der Gehäuse 12 der Umschaltventilbaugruppen 10 eingeführt und in mit Innengewinden versehenen Öffnungen 73a und 73b im Gehäuse 13 der Priorisierungsventilbaugruppe 11 eingedreht (s. Fig. 13).

Die Mantelflächen 46 der Gehäuse 12 der Umschaltventilbaugruppen 10 und die Mantelfläche 47 des Gehäuses 13 der Priorisierungsventilbaugruppe 11 bilden eine gemeinsame Außenfläche des Stapels 4. Das Gehäuse 13 der Priorisierungsventilbaugruppe 11 weist somit im Wesentlichen die gleichen Abmessungen auf wie das Gehäuse 12. Dabei ist es unschädlich, wenn die Höhenabmessung des Gehäuses 13 längs der Stapelachse SA von der Höhenabmessung des Gehäuses 12 abweicht.

An die erste Quellen-Anschlussöffnung 26 der obersten Umschaltventilbaugruppe 10 im Stapel 4 ist eine Sauerstoffflasche 88a unter Zwischenanordnung eines Druckminderers 90a über eine Leitung 92a angeschlossen. Der Druckminderer 90a reduziert den Druck des aus der Sauerstoffflasche 88a austretenden Gases auf einen einheitlichen Druck von beispielsweise 6 bar (600 kPa).

An die zweite Quellen-Anschlussöffnung 54 der obersten Umschaltventilbaugruppe 10 im Stapel 4 ist eine zweite Sauerstoffflasche 88b über einen zweiten Druckminderer 90b mittels einer zweiten Leitung 92b angeschlossen.

Die in Figur 12 nicht erkennbare Ausgabe-Anschlussöffnung 18 der obersten Umschaltventilbaugruppe 10 ist unmittelbar mit der ersten Quellen-Anschlussöffnung 26 als der Folge-Anschlussöffnung 26 der zweiten Umschaltventilbaugruppe 10 des Stapels 4 verbunden, sodass die in Figur 12 obere Umschaltventilbaugruppe 10 des Stapels 4 die Gasquelle für die erste Quellen-Anschlussöffnung 26 der im Stapel 4 unteren Umschaltventilbaugruppe 10 bildet. Auch an die zweite Quellen-Anschlussöffnung 54 der im Stapel 4 unteren Umschaltventilbaugruppe 10 ist eine Sauerstoffflasche angeschlossen, nämlich die Sauerstoffflasche 88c, welche unter Zwischenanordnung eines Druckminderers 90c über die Leitung 92c an die zweite Quellen-Anschlussöffnung 54 angeschlossen ist. Stets liefern die Leitungen 92a, 92b und 92c aufgrund der zwischenangeordneten Druckminderer 90a, 90b und 90c einen konstanten Druck von beispielhaft 6 bar an die jeweiligen Anschlussöffnungen, bis die jeweils zugeordneten Sauerstoffflaschen nahezu vollständig entleert sind.

Die Ausgabe-Anschlussöffnung der in Figur 12 unteren Umschaltventilbaugruppe 12 ist im Stapel 4 mit einer nicht dargestellten ersten Quellen-Anschlussöffnung 27 der Priorisierungsventilbaugruppe 11 gekoppelt, sodass die Umschaltventilbaugruppen 10 die erste Gasquelle der Priorisierungsventilbaugruppe 11 bilden. An eine zweite Quellen-Anschlussöffnung 55 der Priorisierungsventilbaugruppe 11 ist eine Hausleitung 94 einer Klinik K als Gasquelle angeschlossen. Die an sich funktional bekannte Priorisierungsventilbaugruppe 11 ist so gestaltet, dass immer dann wenn eine Hausleitung 94 einer Klinik K als Gasquelle an die Priorisierungsventilbaugruppe 11 angeschlossen ist, das Gas der Hausleitung 94 zur Ausgabe-Anschlussöffnung der Priorisierungsventilbaugruppe 11 geleitet wird, während eine an der ersten Quellen-Anschlussöffnung 27 der Priorisierungsventilbaugruppe 11 angeschlossene Gasquelle erst dann zur Abgabe an die Ausgabe-Anschlussöffnung 19 der Priorisierungsventilbaugruppe 11 freigegeben wird, wenn die Hausleitung 94 von der Priorisierungsventilbaugruppe 11 getrennt ist oder, wider Erwarten, erschöpft sein sollte.

An die Ausgabe-Anschlussöffnung der Priorisierungsventilbaugruppe 11 ist eine Atemgasleitung 96 angeschlossen, welche ein an den Stapel 4 geliefertes Gas zu einer Beatmungsvorrichtung B weiterleitet, welche das Gas wiederum an den Patienten P über eine Schnittstelle 98 ausgibt. Im Beispiel von Figur 12 ist die Schnittstelle 98 als Maske dargestellt. Die Schnittstelle 98 kann jedoch auch eine Nasenbrille sein.

Durch den Stapel 4 kann eine ununterbrochene Belieferung des Patienten P mit gewünschtem Atemgas sichergestellt sein, wobei eine installierte Atemgasquelle in einer Klinik Vorrang vor Gasflaschen als Atemgasquellen hat.

In Figur 13 ist eine Explosionsdarstellung des Stapels 4 dargestellt. Dort ist die erste Quellen-Anschlussöffnung 27 der Priorisierungsventilbaugruppe 11 gezeigt, ebenso der zwischen zwei Betriebsstellungen verlagerbare Priorisierungsventilkolben 77 und die seine Ventilkolbenkavität abschließende Verschlussmutter 85.

Die mit den Durchgangsöffnungen 72a und 72b im Stapeleingriff fluchtenden Öffnungen 73a bzw. 73b weisen Innengewinde auf, in welche die Schrauben 86 eingedreht werden können.

In Figur 14 ist ein Längsschnitt durch den Stapel 4 längs einer die koaxialen Gaslieferbahnen QG1 und AG enthaltenden und zu den Längsabschnitten 44 und 48 parallelen Schnittebene gezeigt.

In Figur 14 ist dargestellt, wie die Gaslieferleitungsabschnitte 20 und 28 der miteinander gestapelten Umschaltventilbaugruppen 10 miteinander fluchten und einen gemeinsamen, kontinuierlichen Gaslieferleitungsbereich 30 bilden.

Auch der Gaslieferleitungsabschnitt 28 der in Figur 14 unteren Umschaltventilbaugruppe 10 fluchtet mit dem ausgehend von der ersten Quellen-Anschlussöffnung 27 der Priorisierungsventilbaugruppe 11 in dessen Gehäuse 13 hinein verlaufenden Gaslieferleitungsabschnitt 29 und bildet mit diesem einen gemeinsamen, kontinuierlichen Gaslieferleitungsbereich 99.

## Patentansprüche

1. Umschaltventilbaugruppe (10), zum Umschalten zwischen zwei Atemgasquellen (88a, 88b, 88c) einer Beatmungsvorrichtung (B), wobei die Umschaltventilbaugruppe (10) umfasst:
- ein Gehäuse (12) mit einer ersten Quellen-Anschlussöffnung (26), mit einer räumlich mit Abstand von der ersten angeordneten zweiten Quellen-Anschlussöffnung (54) und mit einer räumlich mit Abstand von der ersten und der zweiten Quellen-Anschlussöffnung (26, 54) angeordneten Ausgabe-Anschlussöffnung (18),
- eine erste Gaslieferleitung (60), welche die erste Quellen-Anschlussöffnung (26) mit der Ausgabe-Anschlussöffnung (18) verbindet,
- eine zweite Gaslieferleitung (64), welche die zweite Quellen-Anschlussöffnung (54) mit der Ausgabe-Anschlussöffnung (18) verbindet,
- eine im Gehäuse (12) aufgenommene Ventilanordnung (40, 42, 74, 76), welche verstellbar ist zwischen einem ersten Betriebszustand, in welchem sie die erste Gaslieferleitung (60) zur Durchströmung mit Atemgas freigibt und die zweite Gaslieferleitung (64) sperrt, und einem zweiten Betriebszustand, in welchem sie die erste Gaslieferleitung (60) sperrt und die zweite Gaslieferleitung (64) zur Durchströmung mit Atemgas freigibt,
**dadurch gekennzeichnet, dass** die Umschaltventilbaugruppe (10) als modulare Umschaltventilbaugruppe (10) stapelbar ausgebildet ist, indem das Gehäuse (12) einen ersten Außenseitenabschnitt (22) mit einem ersten Stapel-Außenflächenabschnitt (24) und einen relativ zum ersten Außenseitenabschnitt (22) mit Abstand oder/und mit Winkelabstand angeordneten zweiten Außenseitenabschnitt (14) mit einem zum ersten Stapel-Außenflächenabschnitt (24) komplementär ausgebildeten zweiten Stapel-Außenflächenabschnitt (16) aufweist, wobei die Ausgabe-Anschlussöffnung (18) an einem Außenseitenabschnitt (14) aus dem ersten und dem zweiten Außenseitenabschnitt (22, 14) ausgebildet ist, und wobei eine Quellen-Anschlussöffnung (26) aus der ersten und der zweiten Quellen-Anschlussöffnung (26, 54) als Folge-Anschlussöffnung (26) an dem jeweils anderen Außenseitenabschnitt (22) aus dem ersten und dem zweiten Außenseitenabschnitt (14, 22) ausgebildet ist, und zwar derart, dass dann, wenn der erste und der zweite Stapel-Außenflächenabschnitt (16, 24) als Stapel-Außenflächenabschnitte (16, 24) gleichartiger gesonderter modularer Umschaltventilbaugruppen (10) in Stapeleingriff miteinander gedacht sind, von der Auslass- und der Folge-Anschlussöffnung (18, 26) ausgehend sich jeweils ins Gehäuse (12) hinein erstreckenden Gaslieferleitungsabschnitte (20, 28) zu einem kontinuierlichen Gaslieferleitungsbereich (30) verbunden sind.

2. Modulare Umschaltventilbaugruppe (10) nach Anspruch 1,
**dadurch gekennzeichnet, dass** an jedem Außenseitenabschnitt (14, 22) aus dem ersten und dem zweiten Außenseitenabschnitt (14, 22) je wenigstens eine Verbindungsformation (72a, 72b) vorgesehen ist, welche zur Sicherung einer körperlichen Verbindung der modularen Umschaltventilbaugruppe (10) mit einer gleichartigen gesonderten modularen Umschaltventilbaugruppe (10) ausgebildet ist, wobei eine räumliche Relativanordnung zwischen der Folge-Anschlussanordnung (26) und der Verbindungsformation (72a, 72b) in dem die Folge-Anschlussanordnung (26) aufweisenden Außenseitenabschnitt (22) die gleiche räumliche Relativanordnung ist, welche zwischen der Ausgabe-Anschlussöffnung (18) und der Verbindungsformation (72a, 72b) in dem die Ausgabe-Anschlussöffnung (18) aufweisenden Außenseitenabschnitt (14) besteht.

3. Modulare Umschaltventilbaugruppe (10) nach Anspruch 1 oder 2,
**dadurch gekennzeichnet, dass** die jeweils andere Quellen-Anschlussöffnung (54) aus der ersten und der zweiten Quellen-Anschlussöffnung (26, 54), welche nicht die Folge-Anschlussöffnung (26) ist, in einem anderen Außenseitenabschnitt (44) des Gehäuses (12) als dem ersten und dem zweiten Außenseitenabschnitt (14, 22) vorgesehen ist.

4. Modulare Umschaltventilbaugruppe (10) nach einem der vorhergehenden Ansprüche,
**dadurch gekennzeichnet, dass** der erste und der zweite Außenseitenabschnitt (14, 22) auf voneinander weg weisenden, entgegengesetzten Gehäuseseiten ausgebildet sind.

5. Modulare Umschaltventilbaugruppe (10) nach den Ansprüchen 3 und 4,
**dadurch gekennzeichnet, dass** die jeweils andere Quellen-Anschlussöffnung (54) in einem dritten Außenseitenabschnitt (44) ausgebildet ist, wobei der dritte Außenseitenabschnitt (44) den ersten und den zweiten Außenseitenabschnitt (14, 22) miteinander verbindet.

6. Modulare Umschaltventilbaugruppe (10) nach einem der vorhergehenden Ansprüche,
**dadurch gekennzeichnet, dass**
i) der erste Stapel-Außenflächenabschnitt eine Formation aus einem Vorsprung und einer Ausnehmung aufweist und der zweite Stapel-Außenflächenabschnitt die jeweils andere Formation aus einem Vorsprung und einer Ausnehmung komplementär zur Formation des ersten Stapel-Außenflächenabschnitts aufweist, oder dass
ii) der erste Stapel-Außenflächenabschnitt (16) und der zweite Stapel-Außenflächenabschnitt (24) jeweils ebene Stapel-Außenflächenabschnitte (16, 24) sind.

7. Modulare Umschaltventilbaugruppe (10) nach einem der vorhergehenden Ansprüche,
**dadurch gekennzeichnet, dass** der erste und der zweite Außenseitenabschnitt (14, 22) ebene Außenseitenabschnitte (14, 22) sind.

8. Modulare Umschaltventilbaugruppe (10) nach einem der vorhergehenden Ansprüche,
**dadurch gekennzeichnet, dass** das Gehäuse (12) eine parallelepiped-artige Außengestalt aufweist.

9. Modulare Umschaltventilbaugruppe (10) nach einem der vorhergehenden Ansprüche,
**dadurch gekennzeichnet,** die Ventilanordnung (40, 42, 74, 76) einen ersten und einen von dem ersten verschiedenen zweiten Ventilkörper (74, 76) aufweist, wobei der erste und der zweite Ventilkörper (74, 76) jeweils zwischen der ersten und der zweiten Quellen-Anschlussöffnung (26, 54) einerseits und der Ausgabe-Anschlussöffnung (18) andererseits angeordnet sind, wobei der erste Ventilkörper (74) in einer ersten Ventilkörperkavität (40) verlagerbar aufgenommen ist und wobei der zweite Ventilkörper (76) in einer mit Abstand von der ersten Ventilkörperkavität (40) angeordneten zweiten Ventilkörperkavität (42) verlagerbar aufgenommen ist, wobei der erste und der zweite Ventilkörper (74, 76) jeweils verlagerbar sind zwischen einer dem ersten Betriebszustand zugeordneten ersten Betriebsstellung und einer dem zweiten Betriebszustand zugeordneten zweiten Betriebsstellung, wobei die Umschaltventilbaugruppe (10) eine zwischen dem ersten Ventilkörper (74) und der ersten Ventilkörperkavität (40) wirksame erste Dichtungsanordnung (78) aufweist, welche in der ersten Ventilkörperkavität (40) einen ersten Kavitätsbereich (40a) und einen vom ersten verschiedenen zweiten Kavitätsbereich (40b) definiert, wobei die Umschaltventilbaugruppe (10) eine zwischen dem zweiten Ventilkörper (76) und der zweiten Ventilkörperkavität (42) wirksame zweite Dichtungsanordnung (80) aufweist, welche in der zweiten Ventilkörperkavität (42) einen dritten Kavitätsbereich (42a) und einen vom dritten verschiedenen vierten Kavitätsbereich (42b) definiert, wobei ein Kavitätsbereich (40a) aus erstem (40a) und drittem Kavitätsbereich (42a) als erster Liefer-Kavitätsbereich (40a) Teil der ersten Gaslieferleitung (60), aber nicht Teil der zweiten Gaslieferleitung (64) ist, und wobei ein Kavitätsbereich aus zweitem (40b) und viertem Kavitätsbereich (42b) als zweiter Liefer-Kavitätsbereich (40b) Teil der zweiten Gaslieferleitung (64), aber nicht Teil der ersten Gaslieferleitung (60) ist,
wobei die Umschaltventilbaugruppe (10) eine erste Entlüftungsleitung (68) aufweist, welche den ersten Liefer-Kavitätsbereich (40a) mit einer von der Ausgabe-Anschlussöffnung (18) verschiedenen ersten Senken-Anschlussöffnung (68c) verbindet,
wobei die Umschaltventilbaugruppe (10) eine zweite Entlüftungsleitung (70) aufweist, welche den zweiten Liefer-Kavitätsbereich (40b) mit einer von der Ausgabe-Anschlussöffnung (18) verschiedenen zweiten Senken-Anschlussöffnung (70c) verbindet,
wobei jener andere Kavitätsbereich (42a) aus erstem (40a) und drittem Kavitätsbereich (42a), welcher nicht erster Liefer-Kavitätsbereich (40a) ist, als erster Entlüftungs-Kavitätsbereich (42a) Teil der ersten Entlüftungsleitung (68), aber nicht der ersten Gaslieferleitung (60) ist, und wobei jener andere Kavitätsbereich (42b) aus zweitem (40b) und viertem Kavitätsbereich (42b), welcher nicht zweiter Liefer-Kavitätsbereich (40b) ist, als zweiter Entlüftungs-Kavitätsbereich (42b) Teil der zweiten Entlüftungsleitung (70), aber nicht der zweiten Gaslieferleitung (64) ist.

10. Modulare Umschaltventilbaugruppe (10) nach Anspruch 9,
**dadurch gekennzeichnet, dass** der erste Kavitätsbereich (40a) der erste Liefer-Kavitätsbereich (40a) ist, der zweite Kavitätsbereich (40b) der zweite Liefer-Kavitätsbereich (40b) ist, der dritte Kavitätsbereich (42a) der erste Entlüftungs-Kavitätsbereich (42a) ist und der vierte Kavitätsbereich (42b) der zweite Entlüftungs-Kavitätsbereich (42b) ist.

11. Modulare Umschaltventilbaugruppe (10) nach Anspruch 9 oder 10,
**dadurch gekennzeichnet, dass** eine den ersten Entlüftungs-Kavitätsbereich (42a) umgebende Wand einen ersten Entlüftungs-Ventilkörperbereich (76a) umgibt und dass eine den zweiten Entlüftungs-Kavitätsbereich (42b) umgebende Wand einen zweiten Entlüftungs-Ventilkörperbereich (76b) umgibt, wobei der erste Entlüftungs-Ventilkörperbereich (76a) dazu ausgebildet ist, abhängig von der Betriebsstellung des den ersten Entlüftungs-Ventilkörperbereich (76a) aufweisenden Ventilköpers (76), die erste Entlüftungsleitung (68) für eine Gasströmung zu öffnen oder zu schließen, und wobei der zweite Entlüftungs-Ventilkörperbereich (76b) dazu ausgebildet ist, abhängig von der Betriebsstellung des den zweiten Entlüftungs-Ventilkörperbereich (76b) aufweisenden Ventilköpers (76), die zweite Entlüftungsleitung (70) für eine Gasströmung zu öffnen oder zu schließen.

12. Modulare Umschaltventilbaugruppe (10) nach einem der Ansprüche 9 bis 11,
**dadurch gekennzeichnet, dass** eine den ersten Liefer-Kavitätsbereich (40a) umgebende Wand einen ersten Liefer-Ventilkörperbereich (74a) umgibt und dass eine den zweiten Liefer-Kavitätsbereich (40b) umgebende Wand einen zweiten Liefer-Ventilkörperbereich (74b) umgibt, wobei der erste Liefer-Ventilkörperbereich (74a) dazu ausgebildet ist, die erste Gaslieferleitung (60) abhängig von der Betriebsstellung des den ersten Liefer-Ventilkörperbereich (74a) aufweisenden Ventilkörpers (74) für eine Gasströmung zu sperren oder freizugeben, und wobei der zweite Liefer-Ventilkörperbereich (74b) dazu ausgebildet ist, die zweite Gaslieferleitung (64) abhängig von der Betriebsstellung des den zweiten Liefer-Ventilkörperbereich (74b) aufweisenden Ventilkörpers (74) für eine Gasströmung zu sperren oder freizugeben.

13. Modulare Umschaltventilbaugruppe (10) nach Anspruch 11 oder 12,
**dadurch gekennzeichnet, dass** von dem ersten und dem zweiten Ventilkörper (74, 76) nur der den ersten Entlüftungs-Ventilkörperbereich (76a) aufweisende Ventilkörper (76) dazu ausgebildet ist, die erste Entlüftungsleitung (68) für eine Gasströmung zu öffnen oder zu schließen, oder/und dass von dem ersten und dem zweiten Ventilkörper (74, 76) nur der den zweiten Entlüftungs-Ventilkörperbereich (76b) aufweisende Ventilkörper (76) dazu ausgebildet ist, die zweite Entlüftungsleitung (70) für eine Gasströmung zu öffnen oder zu schließen.

14. Umschaltventilanordnung (4), umfassend eine erste Umschaltventilbaugruppe (10) nach einem der vorhergehenden Ansprüche und eine zweite Umschaltventilbaugruppe (10) nach einem der vorhergehenden Ansprüche, wobei der erste Stapel-Außenflächenabschnitt (24) der einen Umschaltventilbaugruppe (10) aus erster und zweiter Umschaltventilbaugruppe (10) mit dem zweiten Stapel-Außenflächenabschnitt (16) der jeweils anderen Umschaltventilbaugruppe (10) aus erster und zweiter Umschaltventilbaugruppe (10) unter Bildung eines über die Auslass-Anschlussanordnung (18) der einen Umschaltventilbaugruppe (10) und die Folge-Anschlussanordnung (26) der anderen Umschaltventilbaugruppe (10) hinweg kontinuierlichen Gaslieferleitungsbereichs (30) in Stapeleingriff miteinander stehen.

15. Beatmungsvorrichtung (B) zur Versorgung eines Patienten (P) mit Atemgas, umfassend einen Atemgaseingang, einen Atemgasausgang, eine Atemgasleitung, welche den Atemgaseingang mit dem Atemgasausgang verbindet, eine Strömungsveränderungsvorrichtung zur Veränderung der pro Zeiteinheit durch den Atemgasausgang strömenden Atemgasmenge oder/und zur Veränderung des Atemgasdrucks am Atemgasausgang und eine Steuereinrichtung zur Steuerung des Betriebs der Strömungsveränderungsvorrichtung, **dadurch gekennzeichnet, dass** stromaufwärts des Atemgaseingangs die Ausgabe-Anschlussöffnung wenigstens einer Umschaltventilbaugruppe (10) nach einem der Ansprüche 1 bis 13 oder einer Umschaltventilanordnung (10) nach Anspruch 14 mit dem Atemgaseingang der Beatmungsvorrichtung (B) gasübertragend verbunden ist.

## Claims

1. Switching-valve assembly (10) for switching between two respiratory gas sources (88a, 88b, 88c) of a respiratory device (B), where the switching-valve assembly (10) comprises:
- A housing (12) with a first source-linking aperture (26), with a second source-linking aperture (54) arranged spatially at a distance from the first one and with an output-linking aperture (18) arranged spatially at a distance from the first and from the second source-linking aperture (26, 54),
- A first gas supply line (60) which connects the first source-linking aperture (26) with the output-linking aperture (18),
- A second gas supply line (64) which connects the second source-linking aperture (54) with the output-linking aperture (18),
- A valve arrangement (40, 42, 74, 76) accommodated in the housing (12) which is displaceable between a first operational state in which it releases the first gas supply line (60) for flow-through of respiratory gas and blocks the second gas supply line (64) and a second operational state in which it blocks the first gas supply line (60) and releases the second gas supply line (64) for flow-through of respiratory gas,
**Characterized in that** the switching-valve assembly (10) is configured stackably as a modular switching-valve assembly (10), **in that** the housing (12) exhibits a first outside section (22) with a first stack exterior surface section (24) and a second outside section (14) arranged at a distance and/or at an angular distance relative to the first outside section (22) with a second stack exterior surface section (16) configured complementarily to the first stack exterior surface section (24), where the output-linking aperture (18) is configured at an outside section (14) out of the first and the second outside section (22, 14) and where a source-linking aperture (26) out of the first and the second source-linking aperture (26, 54) is configured as a follow-up linking aperture (26) at the respective other outside section (22) out of the first and the second outside section (14, 22), namely in such a manner that when the first and the second stack exterior surface sections (16, 24) are conceived as stack exterior surface sections (16, 24) of similar separate modular switching-valve assemblies (10) in stack engagement with one another, gas supply line sections (20, 28) each extending from the outlet- and the follow-up linking apertures (18, 26) into the housing (12) are connected into a continuous gas supply line region (30).

2. Modular switching-valve assembly (10) according to Claim 1,
**Characterized in that** at each outside section (14, 22) out of the first and the second outside section (14, 22) at least one connecting formation (72a, 72b) is provided, which is configured for securing a physical connection of the modular switching-valve assembly (10) with a similar separate modular switching-valve assembly (10), where a relative spatial arrangement between the follow-up linking arrangement (26) and the connecting formation (72a, 72b) in the outside section (22) exhibiting the follow-up linking arrangement (26), is the same relative spatial arrangement which exists between the output-linking aperture (18) and the connecting formation (72a, 72b) in the outside section (14) exhibiting the output-linking aperture (18).

3. Modular switching-valve assembly (10) according to Claim 1 or 2,
**Characterized in that** the respective other source-linking aperture (54) out of the first and the second source-linking aperture (26, 54) which is not the follow-up linking aperture (26), is provided in a different outside section (44) of the housing (12) than the first and the second outside section (14, 22).

4. Modular switching-valve assembly (10) according to one of the preceding Claims,
**Characterized in that** the first and the second outside section (14, 22) are configured on opposite housing sides facing away from one another.

5. Modular switching-valve assembly (10) according to the Claims 3 and 4,
**Characterized in that** the respective other source-linking aperture (54) is configured in a third outside section (44), where the third outside section (44) connects the first and the second outside sections (14, 22) with one another.

6. Modular switching-valve assembly (10) according to one of the preceding Claims,
**Characterized in that**
i) The first stack exterior surface section exhibits a formation out of a projection and a recess and the second stack exterior surface section exhibits the respective other formation out of a projection and a recess complementarily to the formation of the first stack exterior surface section, or that
ii) The first stack exterior surface section (16) and the second stack exterior surface section (24) are each planar stack exterior surface sections (16, 24).

7. Modular switching-valve assembly (10) according to one of the preceding Claims,
**Characterized in that** the first and the second outside section (14, 22) are planar outside sections (14, 22).

8. Modular switching-valve assembly (10) according to one of the preceding Claims,
**Characterized in that** the housing (12) exhibits a parallelepiped-like outer shape.

9. Modular switching-valve assembly (10) according to one of the preceding Claims,
**Characterized in that** the valve arrangement (40, 42, 74, 76) exhibits a first valve body and a second valve body different from the first one (74, 76), where the first and the second valve body (74, 76) are each arranged between the first and the second source-linking apertures (26, 54) on the one hand and the output-linking aperture (18) on the other, where the first valve body (74) is accommodated displaceably in a first valve body cavity (40) and where the second valve body (76) is accommodated displaceably in a second valve body cavity (42) arranged at a distance from the first valve body cavity (40), where the first and the second valve body (74, 76) are each displaceable between a first operational position assigned to the first operational state and a second operational position assigned to the second operational state, where the switching-valve assembly (10) exhibits a first sealing arrangement (78) effective between the first valve body (74) and the first valve body cavity (40) which in the first valve body cavity (40) defines a first cavity region (40a) and a second cavity region (40b) different from the first one, where the switching-valve assembly (10) exhibits a second sealing arrangement (80) effective between the second valve body (76) and the second valve body cavity (42) which in the second valve body cavity (42) defines a third cavity region (42a) and a fourth cavity region (42b) different from the third one, where a cavity region (40a) out of the first (40a) and third cavity region (42a) as first supply cavity region (40a) is part of the first gas supply line (60) but not part of the second gas supply line (64), and where a cavity region out of the second (40b) and fourth cavity region (42b) as second supply cavity region (40b) is part of the second gas supply line (64) but not part of the first gas supply line (60),
Where the switching-valve assembly (10) exhibits a first venting line (68) which connects the first supply cavity region (40a) with a first sink-linking aperture (68c) different from the output-linking aperture (18),
Where the switching-valve assembly (10) exhibits a second venting line (70) which connects the second supply cavity region (40b) with a second sink-linking aperture (70c) different from the output-linking aperture (18),
Where that other cavity region (42a) out of the first (40a) and third cavity region (42a) which is not first supply cavity region (40a) is as first venting cavity region (42a) part of the first venting line (68) but not of the first gas supply line (60), and where that other cavity region (42b) out of the second (40b) and fourth cavity region (42b) which is not second supply cavity region (40b) is as second venting cavity region (42b) part of the second venting line (70) but not of the second gas supply line (64).

10. Modular switching-valve assembly (10) according to Claim 9,
**Characterized in that** the first cavity region (40a) is the first supply cavity region (40a), the second cavity region (40b) is the second supply cavity region (40b), the third cavity region (42a) is the first venting cavity region (42a), and the fourth cavity region (42b) is the second venting cavity region (42b).

11. Modular switching-valve assembly (10) according to Claim 9 or 10,
**Characterized in that** a wall surrounding the first venting cavity region (42a) surrounds a first venting valve body region (76a) and that a wall surrounding the second venting cavity region (42b) surrounds a second venting valve body region (76b), where the first venting valve body region (76a) is configured to open or to close the first venting line (68) to a gas flow depending on the operational position of the valve body (76) exhibiting the first venting valve body region (76a), and where the second venting valve body region (76b) is configured to open or to close the second venting line (70) to a gas flow depending on the operational position of the valve body (76) exhibiting the second venting valve body region (76b).

12. Modular switching-valve assembly (10) according to one of the Claims 9 to 11, **Characterized in that** a wall surrounding the first supply cavity region (40a) surrounds a first supply valve body region (74a) and that a wall surrounding the second supply cavity region (40b) surrounds a second supply valve body region (74b), where the first supply valve body region (74a) is configured to block or release the first gas supply line (60) to a gas flow depending on the operational position of the valve body (74) exhibiting the first supply valve body region (74a), and where the second supply valve body region (74b) is configured to block or release the second gas supply line (64) to a gas flow depending on the operational position of the valve body (74) exhibiting the second supply valve body region (74b).

13. Modular switching-valve assembly (10) according to Claim 11 or 12,
**Characterized in that** out of the first and the second valve body (74, 76) only the valve body (76) exhibiting the first venting valve body region (76a) is configured to open or to close the first venting line (68) to a gas flow, and/or that out of the first and the second valve body (74, 76) only the valve body (76) exhibiting the second venting valve body region (76b) is configured to open or to close the second venting line (70) to a gas flow.

14. Switching-valve arrangement (4), comprising a first switching-valve assembly (10) according to one of the preceding Claims and a second switching-valve assembly (10) according to one of the preceding Claims, where the first stack exterior surface section (24) of the one switching-valve assembly (10) out of first and second switching-valve assembly (10) with the second stack exterior surface section (16) of the respective other switching-valve assembly (10) out of first and second switching-valve assembly (10) are in stack engagement with one another, forming a gas supply line region (30) continuous across the outlet linking arrangement (18) of the one switching-valve assembly (10) and the follow-up linking arrangement (26) of the other switching-valve assembly (10).

15. Respiratory device (B) for supplying a patient (P) with respiratory gas, comprising a respiratory gas entry, a respiratory gas exit, a respiratory gas line which connects the respiratory gas entry with the respiratory gas exit, a flow modification device for modifying the respiratory gas quantity flowing through the respiratory gas exit per unit of time and/or for modifying the respiratory gas pressure at the respiratory gas exit, and a control device for controlling the operation of the flow modification device,
**Characterized in that** upstream of the respiratory gas entry the output-linking aperture of at least one switching-valve assembly (10) according to one of the Claims 1 to 13 or of a switching-valve arrangement (10) according to Claim 14 is connected with the respiratory gas entry of the respiratory device (B) in a gas-conveying manner.

## Revendications

1. Ensemble de soupape de commutation (10) pour commuter entre deux sources de gaz respiratoire (88a, 88b, 88c) d'un dispositif respiratoire (B), l'ensemble de soupape de commutation (10) comprenant :
- un boîtier (12) comportant un premier orifice de connexion de source (26), un deuxième orifice de connexion de source (54) espacé du premier, et un orifice de connexion de sortie (18) espacé des premier et deuxième orifices de connexion de source (26, 54),
- une première conduite d'alimentation en gaz (60) reliant le premier orifice de connexion de source (26) à l'orifice de connexion de sortie (18),
- une deuxième conduite d'alimentation en gaz (64) reliant le deuxième orifice de connexion de source (54) à l'orifice de connexion de sortie (18),
- un ensemble de soupapes (40, 42, 74, 76) logé dans le boîtier (12), lequel ensemble de soupapes est réglable entre un premier état de fonctionnement dans lequel il libère la première conduite d'alimentation en gaz (60) pour qu'elle soit traversée par du gaz respiratoire et bloque la deuxième conduite d'alimentation en gaz (64), et un deuxième état de fonctionnement dans lequel il bloque la première conduite d'alimentation en gaz (60) et libère la deuxième conduite d'alimentation en gaz (64) pour qu'elle soit traversée par du gaz respiratoire,
**caractérisé en ce que** l'ensemble de soupape de commutation (10) est réalisé sous forme d'ensemble de soupape de commutation modulaire (10) empilable, **en ce que** le boîtier (12) présente une première section de côté extérieur (22) avec une première section de surface extérieure d'empilage (24) et une deuxième section de côté extérieur (14) disposée à distance ou/et à distance angulaire par rapport à la première section de côté extérieur (22) avec une deuxième section de surface extérieure d'empilage (16) réalisée de manière complémentaire à la première section de surface extérieure d'empilage (24), dans lequel l'ouverture de connexion de sortie (18) est formée sur une partie de côté extérieur (14) à partir des première et deuxième parties de côté extérieur (22, 14), et dans lequel un orifice de connexion de source (26) parmi les premier et deuxième orifices de connexion de source (26, 54) est formé en tant qu'orifice de connexion de séquence (26) sur l'autre partie latérale extérieure (22) parmi les première et deuxième parties latérales extérieures (14, 22), de telle sorte que, lorsque les première et deuxième parties de surface extérieure d'empilement (16, 24) sont conçues comme des parties de surface extérieure empilées (16, 24) d'ensembles de soupapes de commutation modulaires séparés de même type (10) en engagement empilé l'une avec l'autre, des parties de conduite d'alimentation en gaz (20, 28) s'étendant respectivement dans le boîtier (12) à partir des ouvertures de raccordement de sortie et de suivi (18, 26) sont reliées en une zone de conduite d'alimentation en gaz continue (30).

2. Ensemble de soupape de commutation modulaire (10) selon la revendication 1,
**caractérisé en ce qu**'au moins une formation de liaison (72a, 72b) est prévue sur chaque section latérale extérieure (14, 22) parmi la première et la deuxième section latérale extérieure (14, 22), laquelle est conçue pour assurer une liaison physique de l'ensemble de soupape de commutation modulaire (10) avec un ensemble de soupape de commutation modulaire séparé de même type (10), dans lequel un agencement spatial relatif entre l'agencement de port de séquence (26) et la formation de liaison (72a, 72b) dans la partie latérale extérieure (22) comprenant l'agencement de port de séquence (26) est le même agencement spatial relatif qui existe entre l'orifice de sortie (18) et la formation de liaison (72a, 72b) dans la partie latérale extérieure (14) comprenant l'orifice de sortie (18).

3. Ensemble de soupape de commutation modulaire (10) selon la revendication 1 ou 2,
**caractérisé en ce que** l'autre orifice de connexion de source (54) parmi les premier et deuxième orifices de connexion de source (26, 54), qui n'est pas l'orifice de connexion de séquence (26), est prévu dans une partie latérale extérieure (44) du boîtier (12) autre que les première et deuxième parties latérales extérieures (14, 22).

4. Ensemble de soupape de commutation modulaire (10) selon l'une quelconque des revendications précédentes,
**caractérisé en ce que** les première et deuxième parties latérales extérieures (14, 22) sont formées sur des côtés opposés du boîtier s'écartant l'un de l'autre.

5. Ensemble de soupape de commutation modulaire (10) selon les revendications 3 et 4,
**caractérisé en ce que** l'autre orifice de connexion de source (54) est formé dans une troisième partie latérale extérieure (44), la troisième partie latérale extérieure (44) reliant les première et deuxième parties latérales extérieures (14, 22).

6. Ensemble de soupape de commutation modulaire (10) selon l'une quelconque des revendications précédentes,
**caractérisé en ce que**
i) la première partie de surface extérieure d'empilement comprend une formation d'une saillie et d'un évidement, et la deuxième partie de surface extérieure d'empilement comprend l'autre formation respective d'une saillie et d'un évidement complémentaire à la formation de la première partie de surface extérieure d'empilement, ou que
ii) la première partie de surface extérieure de pile (16) et la seconde partie de surface extérieure de pile (24) sont des parties de surface extérieure de pile planes respectives (16, 24).

7. Ensemble de soupape de commutation modulaire (10) selon l'une quelconque des revendications précédentes,
**caractérisé en ce que** les première et deuxième parties latérales extérieures (14, 22) sont des parties latérales extérieures planes (14, 22).

8. Ensemble de soupape de commutation modulaire (10) selon l'une quelconque des revendications précédentes,
**caractérisé en ce que** le boîtier (12) présente une forme extérieure de type parallélépipédique.

9. Ensemble de soupape de commutation modulaire (10) selon l'une quelconque des revendications précédentes,
**caractérisé en ce que** l'ensemble de soupapes (40, 42, 74, 76) comprend un premier corps de soupape et un deuxième corps de soupape (74, 76) distinct du premier, les premier et deuxième corps de soupape (74, 76) étant respectivement disposés entre les premier et deuxième orifices de connexion de source (26, 54) d'une part et l'orifice de connexion de sortie (18) d'autre part, dans lequel le premier corps de soupape (74) est reçu de manière déplaçable dans une première cavité de corps de soupape (40) et dans lequel le deuxième corps de soupape (76) est reçu de manière déplaçable dans une deuxième cavité de corps de soupape (42) située à distance de la première cavité de corps de soupape (40), le premier et le deuxième corps de soupape (74, 76) pouvant être déplacés respectivement entre une première position de fonctionnement associée au premier état de fonctionnement et une deuxième position de fonctionnement associée au deuxième état de fonctionnement, l'ensemble de soupape de commutation (10) présentant un premier dispositif d'étanchéité (78) agissant entre le premier corps de soupape (74) et la première cavité de corps de soupape (40), définissant dans la première cavité de corps de soupape (40) une première zone de cavité (40a) et une deuxième zone de cavité (40b) différente de la première, l'ensemble de soupape de commutation (10) comprenant un deuxième agencement d'étanchéité (80) agissant entre le deuxième corps de soupape (76) et la deuxième cavité de corps de soupape (42), définissant dans la deuxième cavité de corps de soupape (42) une troisième zone de cavité (42a) et une quatrième zone de cavité (42b) différente de la troisième, dans lequel une zone de cavité (40a) parmi la première (40a) et la troisième (42a) zone de cavité fait partie de la première conduite d'alimentation en gaz (60) en tant que première zone de cavité d'alimentation (40a), mais ne fait pas partie de la deuxième conduite d'alimentation en gaz (64), et dans lequel une zone de cavité parmi la deuxième (40b) et la quatrième (42b) zone de cavité fait partie de la deuxième conduite d'alimentation en gaz (64) en tant que deuxième zone de cavité d'alimentation (40b), mais ne fait pas partie de la première conduite d'alimentation en gaz (60),
dans lequel l'ensemble de soupape de commutation (10) comprend une première conduite de ventilation (68) reliant la première partie de cavité de fourniture (40a) à un premier orifice de raccordement de puits (68c) différent de l'orifice de raccordement de sortie (18),
dans lequel l'ensemble de soupape de commutation (10) comprend une seconde conduite de ventilation (70) reliant la seconde partie de cavité d'alimentation (40b) à un second orifice d'abaissement (70c) différent de l'orifice de sortie (18),
dans lequel l'autre partie de cavité (42a) de la première (40a) et de la troisième (42a) parties de cavité, qui n'est pas la première partie de cavité d'alimentation (40a), fait partie, en tant que première partie de cavité de ventilation (42a), de la première conduite de ventilation (68), mais pas de la première conduite d'alimentation en gaz (60), et dans lequel l'autre partie de cavité (42b) parmi la deuxième (40b) et la quatrième partie de cavité (42b), qui n'est pas la deuxième partie de cavité d'alimentation (40b), fait partie, en tant que deuxième partie de cavité de ventilation (42b), de la deuxième conduite de ventilation (70), mais pas de la deuxième conduite d'alimentation en gaz (64).

10. Ensemble de soupape de commutation modulaire (10) selon la revendication 9,
**caractérisé en ce que** la première zone de cavité (40a) est la première zone de cavité d'alimentation (40a), la deuxième zone de cavité (40b) est la deuxième zone de cavité d'alimentation (40b), la troisième zone de cavité (42a) est la première zone de cavité de purge (42a) et la quatrième zone de cavité (42b) est la deuxième zone de cavité de purge (42b).

11. Ensemble de soupape de commutation modulaire (10) selon la revendication 9 ou 10,
**caractérisé en ce qu**'une paroi entourant la première zone de cavité d'évent (42a) entoure une première zone d'évent de corps de soupape (76a) et en ce qu'une paroi entourant la deuxième zone de cavité d'évent (42b) entoure une deuxième zone d'évent de corps de soupape (76b), la première zone d'évent de corps de soupape (76a) étant adaptée pour être sensible à la position de fonctionnement du corps de soupape (76) comprenant la première zone d'évent de corps de soupape (76a), la première zone d'évent de corps de soupape (68) pour un écoulement de gaz, et dans lequel la deuxième zone d'évent de corps de soupape (76b) est conçue pour ouvrir ou fermer la deuxième conduite de ventilation (70) pour un écoulement de gaz en fonction de la position de fonctionnement du corps de soupape (76) présentant la deuxième zone d'évent de corps de soupape (76b).

12. Ensemble de soupape de commutation modulaire (10) selon l'une quelconque des revendications 9 à 11,
**caractérisé en ce qu'**une paroi entourant la première zone de cavité de fourniture (40a) entoure une première zone de corps de vanne de fourniture (74a) et **en ce qu'**une paroi entourant la deuxième zone de cavité de fourniture (40b) entoure une deuxième zone de corps de vanne de fourniture (74b), la première zone de corps de vanne de fourniture (74a) étant formée pour cela, bloquer ou libérer la première conduite d'alimentation en gaz (60) en fonction de la position de fonctionnement du corps de soupape (74) présentant la première zone de corps de soupape d'alimentation (74a) pour un écoulement de gaz, et la deuxième zone de corps de soupape d'alimentation (74b) étant conçue pour bloquer ou libérer la deuxième conduite d'alimentation en gaz (64) en fonction de la position de fonctionnement du corps de soupape (74) présentant la deuxième zone de corps de soupape d'alimentation (74b) pour un écoulement de gaz.

13. Ensemble de soupape de commutation modulaire (10) selon la revendication 11 ou 12,
**caractérisé en ce que** parmi le premier et le deuxième corps de soupape (74, 76), seul le corps de soupape (76) présentant la première zone d'évent de corps de soupape (76a) est configuré pour ouvrir ou fermer la première conduite de ventilation (68) à un écoulement de gaz, ou/et **en ce que**, parmi le premier et le deuxième corps de soupape (74, 76), seul le corps de soupape (76) présentant la deuxième zone d'évent de corps de soupape (76b) est conçu pour ouvrir ou fermer la deuxième conduite de ventilation (70) pour un écoulement de gaz.

14. Agencement de soupape de commutation (4) comprenant un premier ensemble de soupape de commutation (10) selon l'une quelconque des revendications précédentes et un deuxième ensemble de soupape de commutation (10) selon l'une quelconque des revendications précédentes, dans lequel la première partie de surface extérieure d'empilement (24) d'un ensemble de soupape de commutation (10) parmi les premier et deuxième ensembles de soupape de commutation (10) est reliée à la deuxième partie de surface extérieure d'empilement (16) de l'autre ensemble de soupape de commutation (10) parmi les premier et deuxième ensembles de soupape de commutation (10), en formant un joint de raccordement de sortie et de sortie de l'ensemble de soupape de commutation (10). (18) de l'un des ensembles de soupape de commutation (10) et l'ensemble de raccordement de suivi (26) de l'autre ensemble de soupape de commutation (10) sont en prise d'empilement l'une avec l'autre.

15. Dispositif respiratoire (B) pour alimenter un patient (P) en gaz respiratoire, comprenant une entrée de gaz respiratoire, une sortie de gaz respiratoire, une conduite de gaz respiratoire qui relie l'entrée de gaz respiratoire à la sortie de gaz respiratoire, un dispositif de modification de l'écoulement pour modifier la quantité de gaz respiratoire s'écoulant par unité de temps à travers la sortie de gaz respiratoire ou/et pour modifier la pression de gaz respiratoire à la sortie de gaz respiratoire et un dispositif de commande pour commander le fonctionnement du dispositif de modification de l'écoulement,
**caractérisé en ce qu**'en amont de l'entrée de gaz respiratoire, l'orifice de raccordement de sortie d'au moins un ensemble de soupape de commutation (10) selon l'une des revendications 1 à 13 ou d'un agencement de soupape de commutation (10) selon la revendication 14 est relié en transmission de gaz à l'entrée de gaz respiratoire du dispositif de ventilation (B).
